# EUROPÄISCHE PATENTSCHRIFT

(11) **EP 1 427 701 B1**
(45) Veröffentlichungstag und Bekanntmachung des Hinweises auf die Patenterteilung: **05.05.2010**
(21) Anmeldenummer: 02774560.3
(22) Anmeldetag: 04.09.2002
(51) Int. Cl.: C07D 207/04, C07D 211/14, C07D 209/08, C07D 265/08, C07D 209/44, C07D 215/06, C07C 211/27, C07C 211/28, A01N 43/36, A01N 43/40

(54) **3-AMINOCARBONYL SUBSTITUIERTE BENZOYLCYCLOHEXANDIONE-ALS HERBIZIDE VERWENDBAR**
3-AMINO CARBONYL-SUBSTITUTED BENZOYLCYCLOHEXANEDIONES THAT CAN BE USED AS HERBICIDES
BENZOYLCYCLOHEXANDIONES SUBSTITUEES PAR 3-AMINOCARBONYLE POUVANT ETRE UTILISEES EN TANT QU'HERBICIDES

(30) Priorität: 11.09.2001 DE 10144529
(43) Veröffentlichungstag der Anmeldung: 16.06.2004
(73) Patentinhaber: Bayer CropScience AG, 40789 Monheim (DE)
(72) Erfinder: SEITZ, Thomas, 68519 Viernheim (DE); VAN ALMSICK, Andreas, 61184 Karben (DE); WILLMS, Lothar, 65719 Hofheim (DE); AULER, Thomas, 65812 Bad Soden (DE); BIERINGER, Hermann, 65817 Eppstein (DE); MENNE, Hubert, 65719 Hofheim Ts. (DE)
(86) Internationale Anmeldenummer: PCT/EP2002/009876
(87) Internationale Veröffentlichungsnummer: WO 2003/022810

(56) Entgegenhaltungen:
- EP-A- 0 319 075
- WO-A-01/53275
- WO-A-90/05712
- WO-A-92/07837

## Beschreibung

Die Erfindung betrifft das technische Gebiet der Herbizide, insbesondere das der Herbizide aus der Gruppe der Benzoylcyclohexandione zur selektiven Bekämpfung von Unkräutern und Ungräsern in Nutzpflanzenkulturen, insbesondere in Reiskulturen.

Aus verschiedenen Schriften ist bereits bekannt, daß bestimmte Benzoylcyclohexandione herbizide Eigenschaften besitzen. So sind aus EP-A 0 319 075, WO 92/07837 und WO 96/22958 Benzoylcyclohexandione mit einem HaloalkoxyRest in 3-Position des Phenylrings bekannt. WO 98/42648 nennt Benzoylcyclohexandione, die in 3-Position verschiedene Aminoreste tragen. WO 99/10327, WO 01/53275 und WO 01/32636 beschreiben herbizid wirksame Benzoylcyclohexandione mit einem heterocyclischen Substituenten in 3-Position, der über eine Sauerstoff-Kohlenstoff- Brücke gebunden ist.

Die aus diesen Schriften bekannten Verbindungen zeigen jedoch häufig eine nicht ausreichende herbizide Wirksamkeit. Aufgabe der vorliegenden Erfindung ist daher die Bereitstellung von herbizid wirksamen Verbindungen mit - gegenüber den im dem Stand der Technik offenbarten Verbindungen - verbesserten herbiziden Eigenschaften.

Es wurde nun gefunden, daß Derivate von Benzoylcyclohexandionen, deren Phenylring in 3-Position - über ein Atom aus der Gruppe Sauerstoff, Schwefel und Stickstoff an den Phenylring gebunden - bestimmte Reste aus der Gruppe Aminocarbonylalkyl trägt, als Herbizide besonders gut geeignet sind. Ein Gegenstand der vorliegenden Erfindung sind daher Verbindungen der Formel (I) oder deren Salze 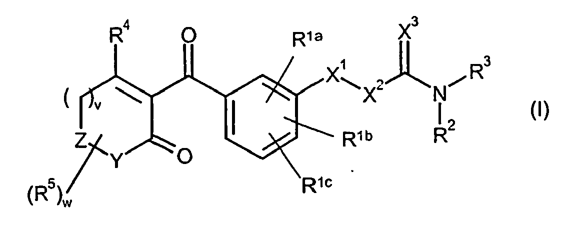 worin
X¹ bedeutet eine divalente Einheit aus der Gruppe O, S(O)ₙ, N-H, N-R²;
X² bedeutet eine geradkettige oder verzweigte durch w Reste aus der Gruppe Halogen, Cyano und Nitro und durch v Reste R² substituierte (C₁-C₆)-Alkylen-, (C₂-C₆)-Alkenylen- oder (C₂-C₆)-Alkinylenkette;
X³ bedeutet Sauerstoff oder Schwefel;
R^{1a}, R^{1b}, R^{1c} bedeuteten unabhängig voneinander Wasserstoff, Mercapto, Nitro, Halogen, Cyano, Thiocyanato, (C₁-C₆)-Alkyl-CO-O, (C₁-C₆)₋Alkyl-S(O)ₙ-O, (C₁-C₆)-Alkyl-S(O)ₙ, Di-(C₁-C₆)-alkyl-NH-SO₂, (C₁-C₆)-Alkyl-SO₂-NH, (C₁-C₆)-Alkyl-NH-CO, (C₁-C₆)-Alkyl-SO₂-[(C₁-C₆)-alkyl]amino, (C₁-C₆)-Alkyl-CO-[(C₁-C₆)-alkyl]amino, 1,2,4-Triazol-1-yl, (C₁-C₆)-Alkyl-O-CH₂, (C₁-C₆)-Alkyl-S(O)ₙ-CH₂, (C₁-C₆)-Alkyl-NH-CH₂, 1,2,4-Triazol-1-yl-CH2, jeweils durch v Reste aus der Gruppe Cyano, Nitro und Halogen substituiertes (C₁-C₆)-Alkyl-(D)ₚ, (C₂-C₆)-Alkenyl-(D)ₚ, (C₂-C₆)-Alkinyl-(D)ₚ, (C₃-C₉)-Cycloalkyl-(D)ₚ, (C₃-C₉)-Cyloalkenyl-(D)ₚ, (C₁-C₆)-Alkyl-Cycloalkyl-(D)ₚ, (C₁-C₆)-Alkyl-Cycloalkenyl-(D)ₚ;
D bedeutet Sauerstoff oder Schwefel;
R², R³ bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₃-C₉)-Cycloalkenyl, (C₁-C₆)-Alkyl-(C₃-C₉)-Cycloalkyl, (C₁-C₆)-Alkyl-(C₃-C₉)-Cycloalkenyl, (C₂-C₆)-Alkenyl-(C₃-C₉)-Cycloalkyl, (C₂-C₆)-Alkenyl-(C₃-C₉)-Cycloalkenyl, (C₂-C₆)-Alkinyl-(C₃-C₉)-Cycloalkyl, (C₂-C₆)-Alkinyl-(C₃-C₉)-Cycloalkenyl, geradkettiges oder verzweigtes [O-C(R⁶)₂]_{w}-[O-C(R⁶)-₂]ₓ-R⁶, (C₁-C₆)-Alkyl-aryl, (C₂-C₆)-Alkenyl-aryl, (C₂-C₆)-Alkinyl-aryl, geradkettiges oder verzweigtes [O-C(R⁶)₂]_{w}-[O-C(R⁶)₂]ₓ-Aryl, wobei die vorstehend genannten kohlenstoffhaltigen Reste durch v Reste aus der Gruppe Cyano, Nitro und Halogen substituiert sind, durch v Reste aus der Gruppe Cyano, Nitro, Halogen, (C₁-C₆)-Alkyl-(D)p und Halogen-(C₁-C₆)-alkyl-(D)ₚ substituiertes Aryl, Heterocyclyl oder Heteroaryl,
   oder
R² und R³ bilden gemeinsam mit dem sie bindenden Stickstoffatom einen 5- oder 6- gliedrigen, gesättigten, teilweise oder vollständig ungesättigten Ring enthaltend m Heteroatome aus der Gruppe Sauerstoff und Stickstoff, an den optional ein Phenylring benzokondensiert ist, wobei der 5- oder 6-gliedrige Ring durch v Reste aus der Gruppe Cyano, Nitro, Halogen, (C₁-C₆)-Alkyl-(D)ₚ und Halogen-(C₁-C₆)-alkyl-(D)ₚ substituiert ist und wobei der ankondensierte Phenylring durch v Reste aus der Gruppe Cyano, Nitro und Halogen substituiert ist;
R⁴ bedeutet OR⁷, (C₁-C₄)-Alkylthio, Halogen-(C₁-C₄)-alkylthio, (C₂-C₄)-Alkenylthio, Halogen-(C₂-C₄)-alkenylthio, (C₂-C₄)-Alkinylthio, Halogen-(C₂-C₄)-alkinylthio, (C₁-C₄)-Alkylsulfinyl, Halogen-(C₁-C₄)-alkylsulfinyl, (C₂-C₄)-Alkenylsulfinyl, Halogen-(C₂-C₄)-alkenylsulfinyl, (C₂-C₄)-Alkinylsulfinyl, Ha)ogen-(C₂-C₄)-alkinylsulfinyl, (C₁-C₄)-Alkylsulfonyl, Halogen-(C₁-C₄)-alkylsulfonyl, (C₂-C₄)-Alkenylsulfonyl, Halogen-(C₂-C₄)-alkenylsulfonyl, (C₂-C₄)-Alkinylsulfonyl, Halogen-(C₂-C₄)-alkinylsulfonyl, Halogen, Cyano, Cyanato, Thiocyanato oder Phenylthio;
R⁵ bedeutet Wasserstoff, Tetrahydropyran-3-yl, Tetrahydropyran-4-yl, Tetrahydrothiopyran-3-yl, (C₁-C₄)-Alkyl, (C₁-C₈)-Cycloalkyl, (C₁-C₄)-Alkoxy, (C₁-C₄)-Alkoxy-(C₁-C₄)-Alkyl, (C₁-C₄)-Alkoxycarbonyl, (C₁-C₄)-Alkylthio, Phenyl, wobei die acht letztgenannten Gruppen durch v Reste aus der Gruppe Halogen, (C₁-C₄)-Alkylthio und (C₁-C₄)-Alkoxy substituiert sind,
   oder
   zwei an einem gemeinsamen C-Atom gebundene Reste R⁵ bilden eine Kette aus der Gruppe OCH₂CH₂O, OCH₂CH₂CH₂O, SCH₂CH₂S und SCH₂CH₂CH₂S, wobei diese durch w Methylengruppen substituiert ist, oder zwei an direkt benachbarten C-Atome gebundene Reste R⁵ bilden mit den sie tragenden C-Atomen einen durch w Reste aus der Gruppe Halogen, (C₁-C₄)-Alkyl, (C₁-C₄)-Alkylthio und (C₁-C₄)-Alkoxy substituierten, 3- bis 6-gliedrigen Ring;
R⁶ bedeutet Wasserstoff, Halogen, Cyano oder Nitro, (C₁-C₄)-Alkyl, Halogen-(C₁-C₄)-alkyl;
R⁷ bedeutet Wasserstoff, (C₁-C₄)-Alkyl, Halogen-(C₁-C₄)-alkyl, (C₁-C₄)-Alkoxy-(C₁-C₄)-alkyl, Formyl, (C₁-C₄)-Alkylcarbonyl, (C₁-C₄)-Alkoxycarbonyl, (C₁-C₄)-Alkylaminocarbonyl, Di-(C₁-C₄)-alkylaminocarbonyl, (C₁-C₄)-Alkylsulfonyl, Halogen-(C₁-C₄)-alkylsulfonyl, Phenyl, Benzoyl oder Phenylsulfonyl, wobei die drei letzgenannten Gruppen durch v Reste aus der Gruppe (C₁-C₄)-Alkyl, Halogen-(C₁-C₄)-alkyl, (C₁-C₄)-Alkoxy, Halogen-(C₁-C₄)-alkoxy, Halogen, Cyano und Nitro substituiert sind;
Y bedeutet eine divalente Einheit aus der Gruppe O, S, N-H, N-(C₁-C₆)-Alkyl, CHR⁵ und C(R⁵)₂;
Z bedeutet eine direkte Bindung oder eine divalente Einheit aus der Gruppe O, S, SO, SO₂ N-H, N-Alkyl, CHR⁶ oder C(R6)₂;

- m und n: bedeuten jeweils unabhängig voneinander 0, 1 oder 2;
- p: bedeutet jeweils unabhängig voneinander 0 oder 1;
- v: bedeutet 0, 1, 2 oder 3;
- w und x: bedeuten jeweils unabhängig voneinander 0,1, 2, 3 oder 4, wobei w und x nicht gleichzeitig null sein sollen.

Für den Fall, daß R⁴ für OH steht, können die erfindungsgemäßen Verbindungen der Formel (I) in Abhängigkeit von äußeren Bedingungen, wie Lösungsmittel und pH-Wert, in unterschiedlichen tautomeren Strukturen auftreten. Je nach Art der Substituenten enthalten die Verbindungen der Formel (I) ein acides Proton, das durch Umsetzung mit einer Base entfernt werden kann. Als Basen eignen sich beispielsweise Hydride, Hydroxide und Carbonate von Alkali- und Erdalkalimetallen, wie Lithium, Natrium, Kalium, Magnesium und Calcium, sowie Ammoniak und organische Amine wie Triethylamin und Pyridin. Solche Salze sind ebenfalls Gegenstand der Erfindung.

In Formel (I) und allen nachfolgenden Formeln können Alkylreste mit mehr als zwei C-Atomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Die Kohlenstoffkette X² kann ebenfalls, je nach Anzahl ihrer C-Atome geradkettig oder verzweigt sein. Die daran gebundenen Reste können sich an beliebiger Position dieser Kette befinden.

Ist eine Gruppe mehrfach durch Reste substituiert, so ist darunter zu verstehen, daß diese Gruppe durch ein oder mehrere gleiche oder verschiedene der genannten Reste substituiert ist.

Cycloalkyl bedeutet ein carbocyclisches, gesättigtes Ringsystem mit drei bis acht C-Atomen, z.B. Cyclopropyl, Cyclopentyl oder Cyclohexyl. Analog bedeutet Cycloalkenyl eine monocyclische Alkenylgruppe mit drei bis acht Kohlenstoffringgliedern, z.B. Cyclopropenyl, Cyclobutenyl, Cyclpentyl und Cyclohexenyl, wobei sich die Doppelbindung an beliebiger Position befinden kann.
Im Falle zusammengesetzter Reste, wie Cycloalkylalkenyl, kann sich der erstgenannte Rest an beliebiger Position des zweitgenannten befinden.

Im Falle einer zweifach substituierten Aminogruppe, wie Dialkylamino, können diese beiden Substituenten gleich oder verschieden sein.

Halogen bedeutet Fluor, Chlor, Brom oder Iod. Halogenalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Halogenalkoxy ist z.B. OCF₃, OCHF₂, OCH₂F, CF₃CF₂O, OCH₂CF₃ und OCH₂CH₂Cl; entsprechendes gilt für Halogenalkenyl und andere durch Halogen substituierte Reste.

Unter dem Begriff Heterocyclyl sind drei- bis sechsgliedrige, gesättigte oder partiell ungesättige mono- oder polycyclische Heterocyclen zu verstehen, die ein bis drei Heteroatome ausgewählt aus einer Gruppe bestehend aus Sauerstoff, Stickstoff und Schwefel enthalten. Die Verknüpfung kann, sofern chemisch möglich an beliebiger Position des Heterocyclus erfolgen. Beispiele dafür sind Oxiranyl, 3-Tetrahydrofuranyl, 2-Tetrahydrothienyl, 3-Tetrahydrothienyl, 1-Pyrrolidinyl, 2-Pyrrolidinyl, 3-Pyrrolidinyl, 3-Isoxazolidinyl, 4-Isoxazolidinyl, 5-Isoxoazolidinyl, 3-Isothioazolidinyl, 4-Isothiazolidinyl, 5-Isothiazolidinyl, 1-Pyrazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxa-diazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydrofur-4-yl, 2,3-Dihydrofur-5-yl, 2,5-Dihydrofur-2-yl, 2,5-Dihydrofur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydro-isoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3-Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydro-oxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, - 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 1-Morpholinyl, 2-Morpholinyl, 3-Morpholinyl, 1-Piperidinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,3-Dithian-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl.

Aryl steht für einen aromatischen mono- oder polycyclischen Kohlenwasserstoffrest, z.B. Phenyl, Naphthyl, Biphenyl und Phenanthryl, vorzugsweise Phenyl. Die Verknüpfung kann grundsätzlich an beliebiger Position von Aryl erfolgen.

Heteroaryl steht für einen aromatischen mono-, bi- oder tricyclischen Rest, der neben Kohlenstoffringgliedern ein bis vier Stickstoffatome oder ein bis drei Stickstoffatome und ein Sauerstoff- oder ein Schwefelatom oder ein Sauerstoff- oder ein Schwefelatom enthält. Die Verknüpfung kann, sofern chemisch möglich, an beliebiger Position von Aryl erfolgen. Beispiele für 5-gliedriges Heteroaryl sind 2-Pyrrolyl, 3-Pyrrolyl, 1-Pyrazolyl, 3-Pyrazolyl, 4-Pyrazolyl, 5-Pyrazolyl, 2-Imidazolyl, 4-Imidazolyl, 1,2,4-Triazol-3-yl, 1,3,4-Triazol-2-yl, 2-Furyl, 3-Furyl, 2-Thienyl,-3-Thienyl, 2-Pyrrolyl, 3-pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl. Beispiele für 6-gliedriges Heteroaryl sind 2-Pyridinyl, 3-Pyridinyl, 4-Pyridinyl, 3-Pyridazinyl, 4-Pyridazinyl, 2-pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl. Beispiele für anneliertes 5-gliedriges Heteroaryl sind Benzothiazol-2-yl und Benzoxazol-2-yl. Beispiele für benzokondensierte 6-gliedriges Heteroaryl sind Chinolin, Isochinolin, Chinazolin und Chinoxalin.

Ist eine Gruppe mehrfach substituiert, so ist darunter zu verstehen, daß bei der Kombination der verschiedenen Substituenten die allgemeinen Grundsätze des Aufbaus chemischer Verbindungen zu beachten sind, d.h. daß nicht Verbindungen gebildet werden, von denen der Fachmann weiß, daß sie chemisch instabil oder nicht möglich sind.

Die Verbindungen der Formel (I) können je nach Art und Verknüpfung der Substituenten als Stereoisomere vorliegen. Sind beispielsweise ein oder mehrere asymmetrische C-Atome vorhanden, so können Enantiomere und Diastereomere auftreten. Stereoisomere lassen sich aus den bei der Herstellung anfallenden Gemischen nach üblichen Trennmethoden, z.B. durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Als vorteilhaft haben sich Verbindungen der Formel (I) herausgestellt, worin R², R³ bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₃-C₉)-Cycloalkenyl, (C₁-C₆)-Alkyl-(C₃-C₉)-Cycloalkyl, (C₁-C₆)-Alkyl-(C₃-C₉)-Cycloalkenyl, (C₂-C₆)-Alkenyl-(C₃-C₉)-Cycloalkyl, (C₂-C₆)-Alkenyl-(C₃-C₉)-Cycloalkenyl, (C₂-C₆)-Alkinyl-(C₃-C₉)-Cycloalkyl, (C₂-C₆)-Alkinyl-(C₃-C₉)-Cycloalkenyl, geradkettiges oder verzweigtes [O-C(R⁶)₂]_{w}-[O-C(R⁶)-₂]ₓ-R⁶, wobei die letzten 12 genannten Reste durch v Reste aus der Gruppe Cyano, Nitro und Halogen substituiert sind, durch v Reste aus der Gruppe Cyano, Nitro, Halogen, (C₁-C₆)-Alkyl-(D)ₚ und Halogen-(C₁-C₆)-alkyl-(D)ₚ substituiertes Aryl,
oder
R² und R³ bilden gemeinsam mit dem sie bindenden Stickstoffatom einen 5- oder 6- gliedrigen, gesättigten, teilweise oder vollständig ungesättigten Ring enthaltend m Heteroatome aus der Gruppe Sauerstoff und Stickstoff, und der durch v Reste aus der Gruppe Cyano, Nitro, Halogen, (C₁-C₆)-Alkyl-(D)ₚ und Halogen-(C₁-C₆)-alkyl-(D)ₚ substituiert ist;
R⁷ bedeutet Wasserstoff, (C₁-C₄)-Alkylsulfonyl, Halogen-(C₁-C₄)-alkylsulfonyl, Phenyl, Benzoyl oder Phenylsulfonyl, wobei die drei letzgenannten Gruppen durch v Reste aus der Gruppe (C₁-C₂)-Alkyl, Halogen-(C₁-C₂)-alkyl, (C₁-C₂)-Alkoxy, Halogen-(C₁-C₂)-alkoxy, Halogen, Cyano und Nitro substituiert sind;
Y bedeutet eine divalente Einheit aus der Gruppe O, N-H, N-(C₁-C₆)-Alkyl, CHR⁵ und C(R⁵)₂, und
Z bedeutet eine divalente Einheit aus der Gruppe O, S, SO₂, (C₁-C₆)-Alkyl, CHR⁶ oder C(R⁶)₂.

Ebenfalls von Vorteil sind Verbindungen der allgemeinen Formel (I),
worin
R² bedeutet (C₁-C₆)-Alkyl und
R³ bedeutet durch v Reste aus der Gruppe Cyano, Nitro und Halogen substituiertes Benzyl, oder
R² und R³ bilden gemeinsam mit dem sie bindenden Stickstoffatom einen durch v Reste aus der Gruppe Cyano, Nitro und Halogen substituierten Rest der Gruppe 2,3-Dihydroindol-1yl, Benzo[c]pyrrolidin-2-yl, 1,2,3,4-Tetrahydrochinolin-1-yl, 1,2,3,4-Tetrahydroisochinolin-2-yl.

Weiterhin von Vorteil sind Verbindungen der allgemeinen Formel (I),
worin
X³ bedeutet Sauerstoff;
R^{1c} bedeutet Wasserstoff, und
R⁶ bedeutet Wasserstoff, (C₁-C₄)-Alkyl oder Halogen-(C₁-C₄)-alkyl.

Bevorzugt sind Verbindungen der allgemeinen Formel (I), worin
X¹ bedeutet Sauerstoff;
R^{1a} und R^{1b} bedeuten jeweils Brom, Chlor, Fluor, Methyl, Methylthio, Methoxy, Methylsulfonyl, Ethylsulfonyl oder Trifluormethyl, und
R², R³ bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₁-C₆)-Alkyl-(C₃-C₉)-cycloalkyl, geradkettiges oder verzweigtes [O-C(R⁶)₂]_{w}-[O-C(R⁶)-₂]ₓ-R⁶, wobei die letzten 6 genannten Reste durch v Reste aus der Gruppe Cyano, Nitro und Halogen substituiert sind, durch v Reste aus der Gruppe Cyano, Nitro, Halogen, (C₁-C₆)-Alkyl-(D)ₚ und Halogen-(C₁-C₆)-alkyl-(D)ₚ substituiertes Aryl,
   oder
R² und R³ bilden gemeinsam mit dem sie bindenden Stickstoffatom einen 5- oder 6- gliedrigen, gesättigten, teilweise oder vollständig ungesättigten Ring enthaltend m Heteroatome aus der Gruppe Sauerstoff und Stickstoff, und der durch v Reste aus der Gruppe Cyano, Nitro, Halogen, (C₁-C₆)-Alkyl-(D)ₚ und Halogen-(C₁-C₆)-alkyl-(D)ₚ substituiert ist.

Bevorzugt sind auch Verbindungen der allgemeinen Formel (I), worin
R⁴ bedeutet OR⁷, (C₁-C₄)-Alkylthio, (C₂-C₄)-Alkenylthio, (C₁-C₄)-Alkylsulfonyl, Halogen, Cyano, Cyanato, Thiocyanato oder Phenylthio und
R⁵ bedeutet Wasserstoff, (C₁-C₄)-Alkyl, (C₁-C₈)-Cycloalkyl, (C₁-C₄)-Alkoxy, (C₁-C₄)-Alkylthio, Phenyl, oder zwei an direkt benachbarten C-Atomen gebundene Reste R⁵ bilden mit den sie tragenden C-Atomen einen substituierten 3- bis 6-gliedrigen Ring.

Besonders bevorzugt sind Verbindungen der allgemeinen Formel (I), worin der Substituent R^{1a} in 2-Position, der Substituent R^{1b} in 4-Position des Phenylrings stehen.

Ganz besonders bevorzugt sind Verbindungen der allgemeinen Formel (I), worin
R⁴ bedeutet OR⁷;
D bedeutet Sauerstoff;
Y und Z bedeuten die Gruppe CH₂, und
v und w bedeuten jeweils unabhängig voneinander 0, 1 oder 2.

Ebenfalls ganz besonders bevorzugt sind Verbindungen der allgemeinen Formel (I), die nicht als Salz vorliegen.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen R⁷ für Wasserstoff steht, können beispielsweise nach der in Schema 1 angegebenen Methode durch basenkatalysierte Umsetzung einer Verbindung der Formel (IIIa), ibn der T für Halogen, Hydroxy oder Alkoxy steht, mit einem Cyclohexandion (II) in Anwesenheit einer Cyanid-Quelle hergestellt werden. Solche Methoden sind beispielsweise in EP-A 0 186 117 beschrieben.

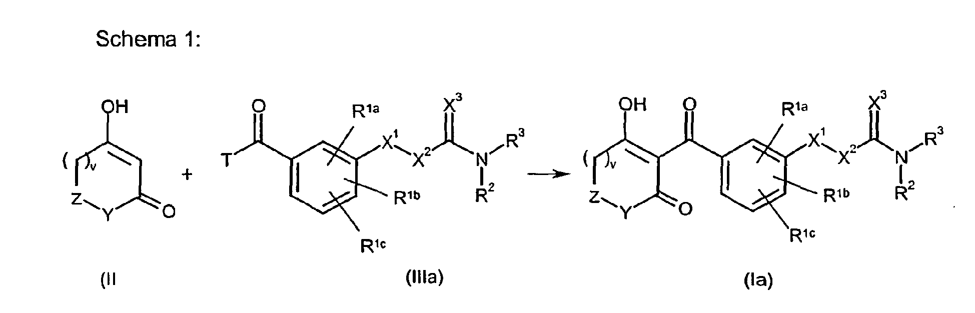

Verbindungen der Formel (IIIa) können beispielsweise nach Schema 2 aus Verbindungen der Formel (IIIb) und (IVa), in der L² für eine Fluchtgruppe wie Halogen, Mesyl, Tosyl oder Triflat steht, gemäß an sich bekannten Methoden hergestellt werden. Solche Methoden sind z.B. aus Houben-Weyl Band 6/3, S. 54 bis 69, Band 9, S. 103 bis 115 und Band 11, S. 97 bekannt.

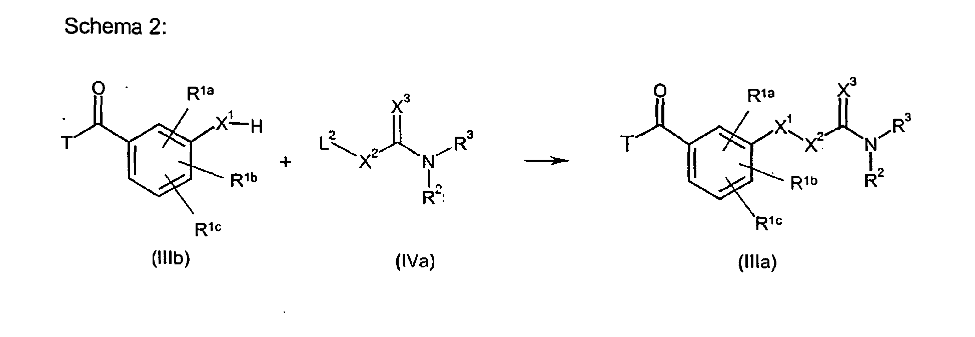

Verbindungen der Formel (IIIa) können gemäß Schema 3 auch durch Umsetzung von Verbindungen der Formel (IIIc), worin L³ für eine Fluchtgruppe wie Triflat oder Nonaflat steht, mit Verbindungen der Formel (IVb) hergestellt werden. Solche Methoden sind z. B. aus WO 98/42648, Houben-Weyl Band 6/3, S. 75 bis 78, Band 9, S. 103 bis 105 bekannt.

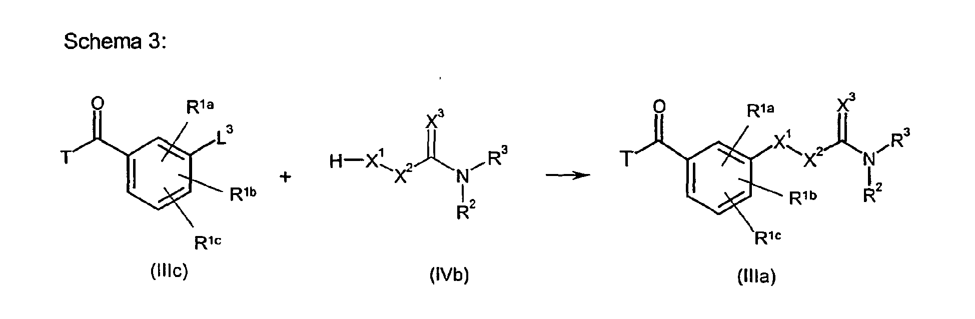

Ebenso können Verbindungen der Formel (IIIa) gemäß Schema 4 durch Umsetzung von Verbindungen der Formel (IIId) mit Verbindungen der Formel (V), worin L⁴ für eine Fluchtgruppe wie Halogen, Mesyl, Tosyl oder Triflat steht, hergestellt werden. Solche Methoden sind z. B. aus Houben-Weyl Band 8, S. 708 bis 709, Band 9, Band E 5/2, S. 998 bis 1001 und 1213 bis 1216 bekannt.

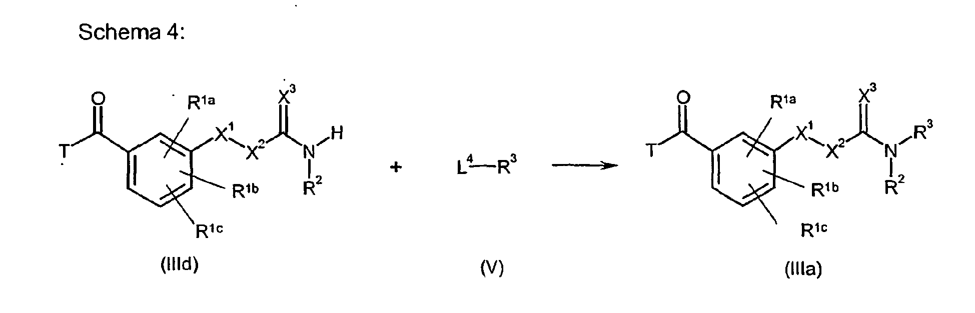

Verbindungen der Formel (IVa) können beispielsweise nach Schema 5 durch Umsetzen von Verbindungen der Formel (VII), in der L³ für eine Gruppe wie Chlor oder Alkoxy und L² für eine Gruppe wie Chlor, Brom, Mesyl oder Tosyl steht, mit Aminen der Formel (VIII) gemäß an sich bekannten Methoden hergestellt werden.
Solche Methoden sind z.B. aus Houben-Weyl Band 8, S. 647 bis 660, Band 11/2, S. 1 bis 73 (insbesondere S. 10 bis 14 und 20 bis 23), Band E 5/2, S. 934 bis 1135 und aus J. Org. Chem. 39 (1974) S. 2607 bis 2612 bekannt.

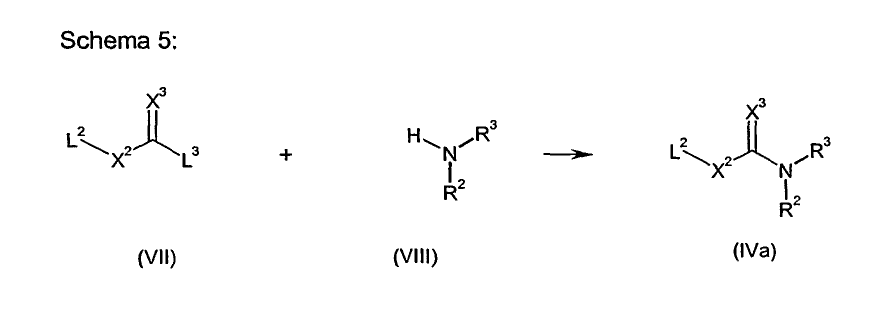

Verbindungen der Formel (IVb) können beispielsweise nach den in US 4,264,520, DE 3 222 229 und J. Med. Chem. 39 (1996) 26, S. 5236 bis 5245 beschriebenen Methoden hergestellt werden.

Erfindungsgemäße Verbindungen der Formel (I), in der R⁴ für andere Reste als Hydroxy steht, können beispielsweise gemäß Schema 4 hergestellt werden. Die darin angegebene Umsetzung einer Verbindung der Formel (la) mit einem Halogenierungsreagenz, wie Oxalylchlorid oder Oxalylbromid, führt zu erfindungsgemäßen Verbindungen der Formel (Ib), die durch Reaktion, gegebenenfalls unter Basenkatalyse, mit Nukleophilen, wie Alkalimetallcyaniden, Alkalimetallcyanaten, Alkalimetallthiocyanaten, Alkylthioalkoholen und Thiophenolen zu weiteren erfindungsgemäßen Verbindungen der Formel (Ic), in der R⁴ für Alkylthio, Halogenalkylthio, Alkenylthio, Halogenalkenylthio, Alkinylthio, Halogenalkinylthio, gegebenenfalls substituiertes Phenylthio, Cyano, Cyanato, Thiocyanato oder OR⁷ steht, umgesetzt werden können. Solche Reaktionen sind beispielsweise beschrieben in Synthesis 12, 1287 (1992). Durch Reaktion mit einem Oxidationsreagenz, wie m-Chlorperoxybenzoesäure, Peroxyessigsäure, Wasserstoffperoxid und Kaliumperoxymonosulfat, werden erfindungsgemäße Verbindungen der Formel (Ic) erhalten, in der R⁴ für Alkylsulfinyl, Halogenalkylsulfinyl, Alkenylsulfinyl, Halogenalkenylsulfinyl, Alkinylsulfinyl, Halogenalkinylsulfinyl, Alkylsulfonyl, Halogenalkylsulfonyl, Alkenylsulfonyl, Halogenalkenylsulfonyl, Alkinylsulfonyl, gegebenenfalls substituiertes Phenylthio oder Halogenalkinylsulfonyl steht. Solche Reaktionen sind beispielsweise beschrieben in J. Org. Chem. 53, 532 (1988), Tetrahedron Lett. 21, 1287 (1981).

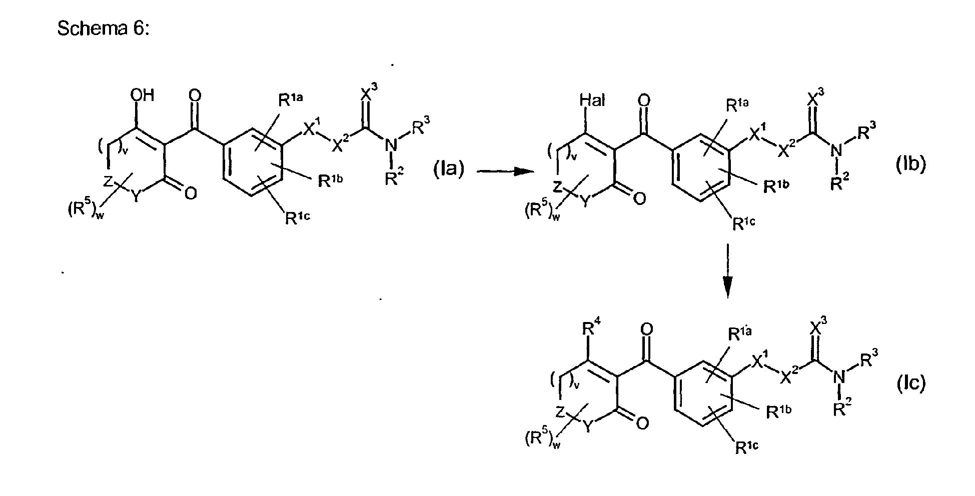

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Amaranthus retroflexus, Avena sp., Echinochloa sp., Cyperus serotinus, Lolium multiflorum, Setaria viridis, Sagittaria pygmaea, Scirpus juncoides, Sinapis sp. und Stellaria media.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Insbesondere weisen sie eine ausgezeichnete Verträglichkeit in Weizen, Mais und Reis auf. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemischphysikalischen Parameter vor-gegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapsel-suspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können z.B. verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäure-ester oder Polyoxethylensorbitanester wie z.B. Polyoxyethylen-sorbitanfettsäureester. Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.
Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 11 th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlomitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031 ); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und - methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1 H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

Die Herstellung der Ausgangsverbindung 2,4-Dibrom-3-hydroxy-benzoesäureethylester erfolgte gemäß US 5,026,896, die Herstellung von 2-Chlor-3-hydroxy-4-ethylsulfonyl-benzoesäure erfolgte gemäß EP-A 0 195 247.
Die Abkürzung RT steht für Raumtemperatur. R_{f} bedeutet Retentionswert.

### Herstellung von 2-(2-Chlor-3-(N,N-diethylaminocarbonyl-methoxy)-4-ethylsulfonyl-benzoyl)-cyclohexan-1,3-dion (Tabellenbeispiel Nr 3.10)

### Schritt 1: 2-Chlor-3-hydroxy-4-ethylsulfonyl-benzoesäuremethylester

33.0 g (124.7 mmol) 2-Chlor-3-hydroxy-4-ethylsulfonyl-benzoesäure wurden in 1300 ml Methanol gelöst. Es wurden 174 ml (3263 mmol) konz. H₂SO₄ zugetropft, und die Mischung wurde 5 h unter Rückfuß erhitzt. Das Reaktionsgemisch wurde eingeengt, und der Rückstand wurde in CH₂Cl₂ aufgenommen. Es wurde mit Waser gewaschen, über Na₂SO₄ getrocknet und vollständig eingeengt. Man erhielt 2-Chlor-3-hydroxy-4-ethylsulfonyl-benzoesäuremethylester als gelbes, zähes Öl.
Ausbeute: 28.23 g (81% der Theorie) R_{f}: (Essigester) 0.45
¹H-NMR: δ [CDCl₃] 1.32 (t, 3H), 3.24 (q, 2H), 3.96 (s, 3H), 7.38 (d, 1H), 7.65.(*d, 1H)

### Schritt 2: 2-Chlor-3-(N,N-diethylaminocarbonyl-methoxy)-4-ethylsulfonyl-benzoesäuremethylester

0.922 g ( 7.2 mmol) K₂CO₃, 0.179 g (1.10 mmol) KI und 0.644 g (4.3 mmol) N,N-Diethylchloracetamid wurden in 30 ml DMF vorgelegt. Bei RT wurden 1.000 g (3.6 mmol) 2-Chlor-3-hydroxy-4-ethylsulfonyl-benzoesäuremethylester zugegeben, und danach für 7 h auf 120°C erhitzt. Dann wurde auf Wasser gegeben und mit Di-isopropylether extrahiert. Die organischen Phasen wurden mit Wasser gewaschen, über Na₂SO₄ getrocknet und vollständig eingeengt. Chromatographie an Kieselgel (Laufmittel: n-Heptan/Essigester 1:1) ergab 2-Chlor-3-(N,N-diethylaminocarbonyl-methoxy)-4-ethylsulfonyl-benzoesäuremethylester als farbloses, zähes Öl.
Ausbeute: 0.50 g (35% der Theorie)
¹H-NMR: δ [CDCl₃] 1.07-1.29 (m, 9H), 3.16 (q, 2H), 3.45 (q, 2H), 3.69 (q, 2H), 3.96 (s, 3H), 4.90 (s, 2H), 7.68 (d, 1H), 7.92 (d, 1H)

### Schritt 3: 2-Chlor-3-(N,N-diethylaminocarbonyl-methoxy)-4-ethylsulfonyl-benzoesäure

0.400 g (1.00 mmol) 2-Chlor-3-(N,N-diethylaminocarbonyl-methoxy)-4-ethylsulfonyl-benzoesäuremethylester wurden in einer Mischung aus 20 ml THF und 20 ml Wasser gelöst und mit 0.082 g (2.00 mmol) NaOH versetzt. Die Mischung wurde 12 h bei Raumtemperatur gerührt und vollständig eingeengt. Der Rückstand wurde in Wasser aufgenommen und mit 6 N HCl versetzt und mit CH₂Cl₂ extrahiert. Trocknen mit Na₂SO₄ und Einengen der organischen Phase ergab 2-Chlor-3-(N, N-diethylaminocarbonyl-methoxy)-4-ethylsulfonyl-benzoesäure farbloses, zähes Öl.
Ausbeute: 0.37 g (99% der Theorie)
¹H-NMR: δ [CDCl₃] 1.13-1.27 (m, 9H), 3.18 (q, 2H), 3.45 (q, 2H), 3.64 (q, 2H), 4.91 (s, 2H), 7.80 (d, 1H), 7.93 (d, 1H)

### Schritt 4: 2-Chlor-3-(N,N-diethylaminocarbonyl-methoxy)-4-ethylsulfonylbenzoesäure-3-oxo-1-cyclohexenyl-ester

0.500 g (1.30 mmol) 2-Chlor-3-(N,N-diethylaminocarbonyl-methoxy)-4-ethylsulfonyl-benzoesäure, 0.163 g (1.50 mmol) Cyclohexan-1,3-dion, 0.259 g (1.30 mmol) N'-(3-Dimethylaminopropyl)-N-ethylcarbodiimid Hydrochlorid und 0.002 g DMAP wurden in 15 ml CH₂Cl₂ 10 h bei RT gerührt. Anschliessend wurde mit CH₂Cl₂ verdünnt und mit 0.5 N HCI, mit Wasser, mit gesättigter NaHCO₃-Lösun und wieder mit Wasser gewaschen. Nach Trocknen der organischen Phasen über Na₂SO₄ und vollständigem Einengen wurde das Produkt in Form eines braunen Harzes erhalten.
Ausbeute: 0.500 g
¹H-NMR: δ [CDCl₃] 1.11-1.27 (m, 9H), 2.13 (m, 2H), 2.45 (m, 2H), 2.68 (m, 2H), 3.17 (q, 2H), 3.45 (q, 2H), 3.70 (q, 2H), 5.29 (s, 2H), 6.07 (m, 1H), 7.80 (d, 1H), 8.00 (d, 1H)

### Schritt 5: 2-(2-Chlor-3-(N,N-diethylaminocarbonyl-methoxy)-4-ethylsulfonyl-benzoyl)-cyclohexan-1,3-dion

0.450 g (ca. 1.00 mmol) 2-Chlor-3-(N,N-diethylaminocarbonyl-methoxy)-4-ethylsulfonylbenzoesäure-3-oxo-1-cyclohexenyl-ester wurden in 10 ml Acetonitril gelöst. Es wurden 3 Tropfen Acetoncyanhydrin sowie 0.164 g (1.60 mmol) NEt₃ zugegeben. Die Mischung wurde 2 h bei RT gerührt, worauf hin 0.019 g (0.30 mmol) KCN zugegeben wurden. Nach weiteren 10 h bei RT wurde eingeengt, in Wasser aufgenommen und mit 6 N HCl versetzt. Danach wurde mit CH₂Cl₂ extrahiert. Nach Trocknen der vereinigten organischen Phasen über Na₂SO₄, vollständigem Einengen und Chromatographie an reversed-phase-Kieselgel (Laufmittel: Acetonitril/Wasser-Gradient) erhielt man (2-Chlor-3-(N,N-diethylaminocarbonyl-methoxy)-4-ethylsulfonyl-benzoyl)-cyclohexan-1,3-dion als farblose, zähes Öl.
Ausbeute: 0.180 g (ca. 40% der Theorie) R_{f}: (Essigester): 0.10
¹H-NMR: δ [CDCl₃] 1.17 (t, 6H), 1.27 (t, 3H), 2.05 (m, 2H), 2.47-2.75 (m, 4H), 3.17 (q, 2H), 3.45 (q, 2H), 3.64 (q, 2H), 4.87 (s, 2H), 7.11 (d, 1H), 7.91(d,1H)

### Herstellung von 2,4-Dibrom-3-(aminocarbonyl-methoxy)-cyclohexan-1,3-dion (Tabellenbeispiel Nr. 3.2)

### Schritt 1: 2,4-Dibrom-3-(aminocarbonyl-methoxy)-benzoesäureethylester

0.853 g (6.20 mmol) K₂CO₃, 0.154g (0.90 mmol) KI und 1.000 g (3.10 mmol) 2,4-Dibrom-3-hydroxy-benzoesäureethylester wurden in 15 ml DMF vorgelegt. Bei RT wurden 0.346 g (3.70 mmol) Chloracetamid zugegeben, und die Mischung wurde für 6 h auf 120°C erhitzt. Anschliessend wurde auf Wasser gegeben und mit Diisopropylether extrahiert. Die vereinigten organischen Phasen wurden mit Wasser gewaschen, über Na₂SO₄ getrocknet und vollständig eingeengt. Trocknen im Ölpumpenvakuum ergab 2,4-Dibrom-3-(aminocarbonyl-methoxy)-benzoesäureethylester als braunes Öl.
Ausbeute: 0.80 g (68% der Theorie)
¹H-NMR: δ [CDCl₃] 1.20 (t, 3H), 4.20 (q, 2H), 4.39 (s, 2H), 7.43 (d, 1H), 7.60 (d, 1H)

### Schritt 2: 2,4-Dibrom-3-(aminocarbonyl-methoxy)-benzoesäure

0.700 g (1.80 mmol) 2,4-Dibrom-3-(aminocarbonyl-methoxy)-benzoesäureethylester wurden in 15 ml THF und 15 ml Wasser gelöst und mit 0.147 g (3.70 mmol) NaOH versetzt. Die Mischung wurde 12 h bei RT gerührt und vollständig eingeengt. Der Rückstand wurde in Wasser aufgenommen und mit 6 N HCl versetzt. Der ausgefallene Niederschlag wurde abgesaugt und getrocknet. Es wurde 2,4-Dibrom-3-(aminocarbonyl-methoxy)-benzoesäure als weißer Feststoff erhalten.
Ausbeute: 0.560 g (88% der Theorie)
¹H-NMR: [d6-DMSO] 4.51 (s, 2H), 7.43 (d, 1H), 7.75 (d, 1H)

### Schritt 3: 2,4-Dibrom-3-(aminocarbonyl-methoxy)-benzoesäure-3-oxo-1-cyclohexenyl-ester

0.400 g (1.10 mmol) 2,4-Dibrom-3-(aminocarbonyl-methoxy)-benzoesäure, 0.140 g (1.20 mmol) Cyclohexan-1,3-dion, 0.222 g (1.10 mmol) N'-(3-Dimethylaminopropyl)-N-ethylcarbodiimid Hydrochlorid und 0.001 g DMAP wurden in 15 ml CH₂Cl₂ 14 h bei RT gerührt. Anschliessend wurde mit CH₂Cl₂ verdünnt und mit 0.5 N HCl, mit Wasser, mit gesättigter NaHCO₃-Lösung und wieder mit Wasser gewaschen. Nach Trocknen der vereinigten organischen Phasen über Na₂SO₄ und vollständigem Einengen wurde 2,4-Dibrom-3-(aminocarbonyl-methoxy)-benzoesäure-3-oxo-1-cyclohexenyl-ester als gelbes Harz erhalten, das für die Folgeumsetzung ausreichend rein war.
Ausbeute: 0.300 g
¹H-NMR: δ [CDCl₃] 2.13 (m, 2H), 2.47 (m, 2H), 2.69 (m, 1H), 4.71 (s, 2H), 6.05 (m, 1H), 7.61 (d, 1H), 7.67 (d, 1H)

### Schritt 4: 2,4-Dibrom-3-(aminocarbonyl-methoxy)-cyclohexan-1,3-dion

0.300 g (ca. 0.70 mmol) 2,4-Dibrom-3-(aminocarbonyl-methoxy)-benzoesäure-3-oxo-1-cyclohexenyl-ester wurden in 10 ml Acetonitril gelöst. Es wurden 3 Tropfen Acetoncyanhydrin sowie 0.115 g (1.10 mmol) NEt₃ zugegeben. Die Mischung wurde 2 h bei RT gerührt, woraufhin 0.005 g (0.10 mmol) KCN zugegeben wurden. Nach weiteren 10 h bei RT wurde vollständig eingeengt, der Rückstand in Wasser aufgenommen und mit 6 N HCl versetzt. Anschliessend wurde mit CH₂Cl₂ extrahiert. Nach Trocknen der vereinigten organischen Phasen über Na₂SO₄, vollständigem Einengen und Chromatographie an reversed-phase-Kieselgel (Laufmittel: Acetonitril/Wasser-Gradient) erhielt man (2,4-Dibrom-3-(aminocarbonyl-methoxy)-cyclohexan-1,3-dion als farbloses, zähes Öl.
Ausbeute: 0.040 g (ca. 13% der Theorie) R_{f}: (Essigester): 0.45
¹H-NMR: δ [CDCl₃] 2.06 (m, 2H), 2.43 (m, 2H), 2.80 (d, 2H), 4.68 (s, 2H), 6.85 (d, 1H), 7.60 (d, 1H)

Die in nachfolgenden Tabellen aufgeführten Beispiele wurden analog oben genannten Methoden hergestellt beziehungsweise sind analog den oben genannten Methoden erhältlich.

Die hier verwendeten Abkürzungen bedeuten:

| | | | | | | | |
|---|---|---|---|---|---|---|---|
| Bu | = Butyl | c | = cyclo | Et | = Ethyl | Me | = Methyl |
| Ph | = Phenyl | Pr | = Propyl | Fp. | = Festpunkt | EE | = Essigester |

**Tabelle 1: Erfindungsgemäße Verbindungen der allgemeinen Formel (I), worin die Substituenten und Symbole folgende Bedeutungen haben:**

| | | | | |
|---|---|---|---|---|
| | R^{1a} = Cl | R^{1b} = SO₂Me | | R^{1c} = H |
| | R⁴ = OH | X³ = O | | Y = CH₂ |
| | Z = CH₂ | | | |
| 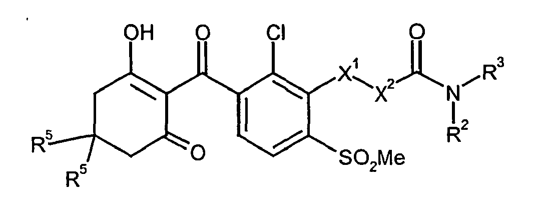 | | | | |

| **Nr.** | **X¹-X²** | **-NR²R³** | **R⁵** | **Physikal. Daten** |
|---|---|---|---|---|
| 1.1 | -OCH₂- | -NH₂ | H | |
| 1.2 | -OCH₂- | -NH₂ | Me | |
| 1.3 | -OCH₂- | -NHMe | H | |
| 1.4 | -OCH₂- | -NHEt | H | |
| 1.5 | -OCH₂- | -NH(n-Pr)H | H | |
| 1.6 | -OCH₂- | -N H (i-Pr) | H | |
| 1.7 | -OCH₂- | -NH(CH₂CH=CH₂) | H | |
| 1.8 | -OCH₂- | -NH(c-Pr) | H | |
| 1.9 | -OCH₂- | -NH(CH₂-c-Pr) | H | |
| 1.10 | -OCH₂- | -NH(c-Bu) | H | |
| 1.11 | -OCH₂- | -NH(c-Pentyl) | Me | |
| 1.12 | -OCH₂- | -NH(c-Hexyl) | H | |
| 1.13 | -OCH₂- | -NH(Ph) | H | |
| 1.14 | -OCH₂- | -NH(2,4-CI₂Ph) | H | |
| 1.15 | -OCH₂- | -N(Me)₂ | H | R_{f} (EE): 0.07 |
| 1.16 | -OCH₂- | -N(Et)₂ | H | R_{f} (EE): 0.39 |
| 1.17 | -OCH₂- | -N(i-Pr)₂ | H | R_{f} (EE): 0.11 |
| 1.18 | -OCH₂- | -N(Me)OMe | H | |
| 1.19 | -OCH₂- | -N(CH₂CH=CH₂)₂ | H | R_{f} (EE): 0.33 |
| 1.20 | -OCH₂- | -N(c-Pr)₂ | H | |
| 1.21 | -OCH₂- | -N(CH₂-c-Pr)₂ | H | |
| 1.22 | -OCH₂- | -N(c-Bu)₂ | H | |
| 1.23 | -OCH₂- | -N(c-Pentyl)₂ | H | |
| 1.24 | -OCH₂- | -N(c-Hexyl)₂ | H | |
| 1.25 | -OCH₂- | -N(Et)Ph | H | |
| 1.26 | -OCH₂- | -NPh₂ | H | |
| 1.27 | -OCH₂- | -N(2,4-Cl₂-Ph)₂ | H | |
| 1.28 | -OCH₂- | -N(Ph)Me | H | R_{f} (EE): 0.08 |
| 1.29 | -OCH₂- | -N(Ph)i-Pr | H | R_{f} (EE): 0.09 |
| 1.30 | -OCH₂- | 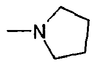 | H | R_{f} (EE): 0.04 |
| 1.31 | -OCH₂- | 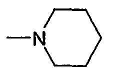 | H | R_{f} (EE): 0.07 |
| 1.32 | -OCH₂- | 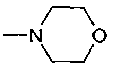 | H | |
| 1.33 | -OCH₂CH₂- | -NH₂ | H | |
| 1.34 | -OCH₂CH₂- | -NHMe | H | |
| 1.35 | -OCH₂CH₂- | -NHEt | H | |
| 1.36 | -OCH₂CH₂- | -NH(n-Pr) | H | |
| 1.37 | -OCH₂CH₂- | -NH(i-Pr) | H | |
| 1.38 | -OCH₂CH₂- | -NH(CH₂CH=CH₂) | H | |
| 1.39 | -OCH₂CH₂- | -NH(c-Pr) | H | |
| 1.40 | -OCH₂CH₂- | -NH(CH₂-c-Pr) | H | |
| 1.41 | -OCH₂CH₂- | -NH(c-Bu) | H | |
| 1.42 | -OCH₂CH₂- | -NH(c-Pentyl) | Me | |
| 1.43 | -OCH₂CH₂- | -NH(c-Hexyl) | H | |
| 1.44 | -OCH₂CH₂- | -NH(Ph) | H | |
| 1.45 | -OCH₂CH₂- | -NH(2,4-Cl₂Ph) | H | |
| 1.46 | -OCH₂CH₂- | -NMe₂ | H | |
| 1.47 | -OCH₂CH₂- | -NEt₂ | H | |
| 1.48 | -OCH₂CH₂- | -N(i-Pr)₂ | H | |
| 1.49 | -OCH₂CH₂- | -N(Me)OMe | H | |
| 1.50 | -OCH₂CH₂- | -N(CH₂CH=CH₂)₂ | H | |
| 1.61 | -OCH₂CH₂- | -N(c-Pr)₂ | H | |
| 1.62 | -OCH₂CH₂- | -N(CH₂-c-Pr)₂ | H | |
| 1.63 | -OCH₂CH₂- | -N(c-Bu)₂ | H | |
| 1.64 | -OCH₂CH₂- | -N(c-Pentyl)₂ | H | |
| 1.65 | -OCH₂CH₂- | -N(c-Hexyl)₂ | H | |
| 1.66 | -OCH₂CH₂- | -N(Et)Ph | H | |
| 1.67 | -OCH₂CH₂- | -NPh₂ | H | |
| 1.68 | -OCH₂CH₂- | -N(2,4-Cl₂-Ph)₂ | H | |
| 1.69 | -OCH₂CH₂- | -N(Ph)Me | H | |
| 1.70 | -OCH₂CH₂- | -N(Ph)Me | Me | |
| 1.71 | -OCH₂CH₂- | -N(Ph)i-Pr | H | |
| 1.72 | -OCH₂CH₂- | 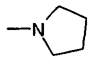 | H | |
| 1.73 | -OCH₂CH₂- | 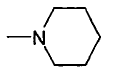 | H | |
| 1.74 | -OCH₂CH₂- | 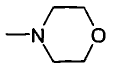 | H | |
| 1.75 | -OCH₂CH=CH- | -NH₂ | H | |
| 1.76 | -OCH₂CH=CH- | -NHMe | H | |
| 1.77 | -OCH₂CH=CH- | -NHEt | H | |
| 1.78 | -OCH₂CH=CH- | -NH(n-Pr) | H | |
| 1.79 | -OCH₂CH=CH- | -NH(i-Pr) | H | |
| 1.80 | -OCH₂CH=CH- | -NH(CH₂CH=CH₂) | H | |
| 1.81 | -OCH₂CH=CH- | -NH(c-Pr) | H | |
| 1.82 | -OCH₂CH=CH- | -NH(CH₂-c-Pr) | H | |
| 1.83 | -OCH₂CH=CH- | -NH(c-Bu) | H | |
| 1.84 | -OCH₂CH=CH- | -NH(c-Pentyl) | Me | |
| 1.85 | -OCH₂CH=CH- | -NH(c-Hexyl) | H | |
| 1.86 | -OCH₂CH=CH- | -NH(Ph) | H | |
| 1.87 | -OCH₂CH=CH- | -NH(2,4-Cl₂Ph) | H | |
| 1.88 | -OCH₂CH=CH- | -N(Me)₂ | H | |
| 1.89 | -OCH₂CH=CH- | -N(Et)₂ | H | |
| 1.90 | -OCH₂CH=CH- | -N(i-Pr)₂ | H | |
| 1.91 | -OCH₂CH=CH- | -N(Me)OMe | H | |
| 1.92 | -OCH₂CH=CH- | -N(CH₂CH=CH₂)₂ | H | |
| 1.93 | -OCH₂CH=CH- | -N(c-Pr)₂ | H | |
| 1.94 | -OCH₂CH=CH- | -N(CH₂-c-Pr)₂ | H | |
| 1.95 | -OCH₂CH=CH- | -N(c-Bu)₂ | H | |
| 1.96 | -OCH₂CH=CH- | -N(c-Pentyl)₂ | H | |
| 1.97 | -OCH₂CH=CH- | -N(c-Hexyl)₂ | H | |
| 1.98 | -OCH₂CH=CH- | -N(Et)Ph | H | |
| 1.99 | -OCH₂CH=CH- | -NPh₂ | H | |
| 1.100 | -OCH₂CH=CH- | -N(2,4-Cl₂-Ph)₂ | H | |
| 1.101 | -OCH₂CH=CH- | -N(Ph)Me | H | |
| 1.102 | -OCH₂CH=CH- | -N(Ph)i-Pr | H | |
| 1.103 | -OCH₂CH=CH- | 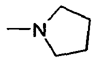 | H | |
| 1.104 | -OCH₂CH=CH- | 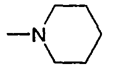 | H | |
| 1.105 | -OCH₂CH=CH- | 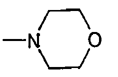 | H | |
| 1.106 | -OCH₂CH=CH- | 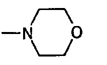 | Me | |
| 1.107 | -OCH₂C≡C- | -NH₂ | H | |
| 1.108 | -OCH₂C≡C- | -NHMe | H | |
| 1.109 | -OCH₂C≡C- | -NHEt | H | |
| 1.110 | -OCH₂C≡C- | -NH(n-Pr) | H | |
| 1.111 | -OCH₂C≡C- | -NH(i-Pr) | H | |
| 1.112 | -OCH₂C≡C- | -NH(CH₂CH=CH₂) | H | |
| 1.113 | -OCH₂C≡C- | -NH(c-Pr) | H | |
| 1.114 | -OCH₂C≡C- | -NH(CH₂-c-Pr) | H | |
| 1.115 | -OCH₂C≡C- | -NH(c-Bu) | H | |
| 1.116 | -OCH₂C≡C- | -NH(c-Pentyl) | H | |
| 1.117 | -OCH₂C≡C- | -NH(c-Hexyl) | H | |
| 1.118 | -OCH₂C≡C- | -NH(Ph) | H | |
| 1.119 | -OCH₂C≡C- | -NH(2,4-CI₂Ph) | H | |
| 1.120 | -OCH₂C≡C- | -NMe₂ | H | |
| 1.121 | -OCH₂C≡C- | -NEt₂ | H | |
| 1.122 | -OCH₂C≡C- | -N(i-Pr)₂ | H | |
| 1.123 | -OCH₂C≡C- | -N(Me)OMe | Me | |
| 1.124 | -OCH₂C≡C- | -N(CH₂CH=CH₂)₂ | H | |
| 1.125 | -OCH₂C≡C- | -N(c-Pr)₂ | H | |
| 1.126 | -OCH₂C≡C- | -N(CH₂-c-Pr)₂ | H | |
| 1.127 | -OCH₂C≡C- | -N(c-Bu)₂ | H | |
| 1.128 | -OCH₂C≡C- | -N(c-Pentyl)₂ | H | |
| 1.129 | -OCH₂C≡C- | -N(c-Hexyl)₂ | H | |
| 1.130 | -OCH₂C≡C- | -N(Et)Ph | H | |
| 1.131 | -OCH₂C≡C- | -NPh₂ | H | |
| 1.132 | -OCH₂C≡C- | -N(2,4-Cl₂₇Ph)₂ | H | |
| 1.133 | -OCH₂C≡C- | -N(Ph)Me | H | |
| 1.134 | -OCH₂C≡C- | -N(Ph)i-Pr | H | |
| 1.135 | -OCH₂C≡C- | 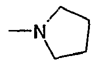 | H | |
| 1.136 | -OCH₂C≡C- | 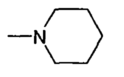 | H | |
| 1.137 | -OCH₂C≡C- | 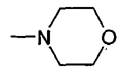 | H | |
| 1.138 | -OCH₂- | -NEt₂ | Me | R_{f} (EE): 0.60 |
| 1.139 | -OCH₂- | -NMeEt | H | R_{f} (EE): 0.21 |
| 1.140 | -OCH₂- | -NMe(CH₂)Ph | H | R_{f} (EE): 0.20 |
| 1.141 | -OCH₂- | 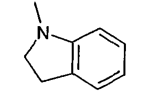 | H | R_{f} (EE): 0.12 |
| 1.142 | -OCH₂- | 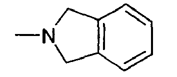 | H | R_{f} (EE): 0.04 |
| 1.143 | -OCH₂- | 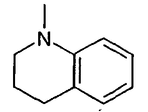 | H | R_{f} (EE): 0.11 |
| 1.144 | -OCH₂- | 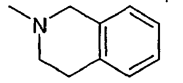 | H | R_{f} (EE): 0.07 |

**Tabelle 2: Erfindungsgemäße Verbindungen der Formel (I), worin die Substituenten und Symbole folgende Bedeutungen haben:**

| | | | | |
|---|---|---|---|---|
| | R^{1a} = CI | R^{1b} = SO₂Me | | R^{1c} = H |
| | X³ = O | Y = CH₂ | | Z = CH₂ |
| 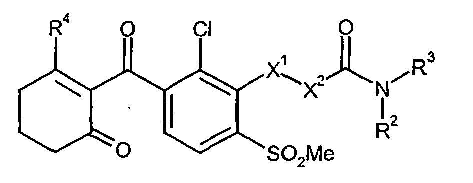 | | | | |

| **Nr.** | **X¹-X²** | **-NR²R³** | **R⁴** | **Physikal. Daten** |
|---|---|---|---|---|
| 2.1 | -OCH₂- | -NH₂ | O-Ph | |
| 2.2 | -OCH₂- | -NH₂ | S-Allyl | |
| 2.3 | -OCH₂- | -NH(i-Pr) | O-Et | |
| 2.4 | -OCH₂- | -NH(CH₂CH=CH₂) | O-Ph | |
| 2.5 | -OCH₂- | -NH(c-Pr) | S-Ph | |
| 2.6 | -OCH₂- | -NH(c-Hexyl) | O-Ph | |
| 2.7 | -OCH₂- | -NH(2,4-Cl₂Ph) | S-Ph | |
| 2.8 | -OCH₂- | -NMe₂ | S-h | |
| 2.9 | -OCH₂- | -NEt₂ | S-Allyl | |
| 2.10 | -OCH₂- | -N(CH₂CH=CH₂)₂ | O-Et | |
| 2.11 | -OCH₂- | -N(Et)v-Hexyl | O-Et | |
| 2.12 | -OCH₂- | -N(Ph)i-Pr | S-Ph | |
| 2.13 | -OCH₂- | 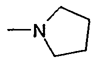 | O-Ph | |
| 2.14 | -OCH₂- | 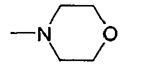 | S-Ph | |
| 2.15 | -OCH₂CH₂- | -NH₂ | O-Ph | |
| 2.16 | -OCH₂CH₂- | -NMe₂ | S-Allyl | |
| 2.17 | -OCH₂CH₂- | -NH(CH₂-c-Pr) | S-Ph | |
| 2.18 | -OCH₂CH₂- | -N(CH₂CH=CH₂)₂ | O-Et | |
| 2.19 | -OCH₂CH₂- | 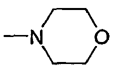 | S-Ph | |
| 2.20 | -OCH₂CH=CH- | -NH₂ | O-Ph | |
| 2.21 | -OCH₂CH=CH- | -NMe₂ | S-Allyl | |
| 2.22 | -OCH₂CH=CH- | -NH(CH₂-c-Pr) | S-Ph | |
| 2.23 | -OCH₂CH=CH- | -N(CH₂CH=CH₂)₂ | O-Et | |
| 2.24 | -OCH₂CH=CH- | 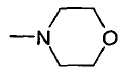 | S-Ph | |
| 2.25 | -OCH₂C≡C- | -NH₂ | O-Ph | |
| 2.26 | -OCH₂C≡C- | -NMe₂ | S-Allyl | |
| 2.27 | -OCH₂C≡C- | -NH(CH₂-c-Pr) | S-Ph | |
| 2.28 | -OCH₂C≡C- | -N(CH₂CH=CH₂)₂ | O-Et | |
| 2.29 | -OCH₂C≡C- | 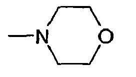 | S-Ph | |

**Tabelle 3: Erfindungsgemäße Verbindungen der Formel (I), worin die Substituenten und Symbole folgende Bedeutungen haben:**

| | | | | | |
|---|---|---|---|---|---|
| | R^{1c} = H | | X³ = O | | R⁴ = OH |
| | Y = CH₂ | | Y = CH₂ | | Z = CH₂ |
| 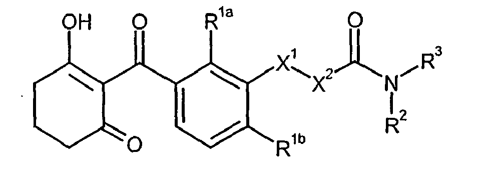 | | | | | |

| **Nr.** | **R^{1a}** | **R^{1b}** | **X¹-X²** | **-NR²R³** | **Physikal. Daten** |
|---|---|---|---|---|---|
| 3.1 | Cl | Cl | -OCH₂- | -NH₂ | R_{f} (EE): 0.04 |
| 3.2 | Br | Br | -OCH₂- | -NH₂ | R_{f} (EE): 0.04 |
| 3.3 | Cl | Cl | -OCH₂- | -NHMe | |
| 3.4 | Br | Br | -OCH₂- | -NHMe | |
| 3.5 | Cl | Cl | -OCH₂- | -NHEt | |
| 3.6 | Me | SO₂Me | -OCH₂- | -NHEt | |
| 3.7 | Cl | Cl | -OCH₂- | -NMe₂ | R_{f} (EE): 0.20 |
| 3.8 | Cl | SO₂Et | -OCH₂- | -NMe₂ | R_{f} (EE): 0.08 |
| 3.9 | Cl | Cl | -OCH₂- | -NEt₂ | R_{f} (EE): 0.45 |
| 3.10 | Cl | SO₂Et | -OCH₂- | -NEt₂ | R_{f} (EE): 0.10 |
| 3.11 | Br | Br | -OCH₂- | -NH(CH₂-c-Pr) | |
| 3.12 | Cl | SO₂Et | -OCH₂- | 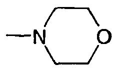 | |
| 3.13 | Cl | Cl | -OCH₂- | -N(Me)OMe | |
| 3.14 | Me | SO₂Et | -OCH₂- | -N(Me)OMe | |
| 3.15 | Br | Br | -OCH₂- | -N(Me)Ph | |
| 3.16 | Cl | Cl | -OCH₂CH₂- | -NH₂ | |
| 3.17 | Br | Br | -OCH₂CH₂- | -NH₂ | |
| 3.18 | Cl | Cl | -OCH₂CH₂- | -NHMe | |
| 3.19 | Br | Br | -OCH₂CH₂- | -NHMe | |
| 3.20 | Cl | Cl | -OCH₂CH₂- | -NHEt | |
| 3.21 | Me | SO₂Me | -OCH₂CH₂- | -NHEt | |
| 3.22 | Cl | Cl | -OCH₂CH₂- | -NMe₂ | |
| 3.23 | Cl | SO₂Et | -OCH₂CH₂- | -NMe₂ | |
| 3.24 | Cl | Cl | -OCH₂CH₂- | -NEt₂ | |
| 3.25 | Cl | SO₂Et | -OCH₂CH₂- | -NEt₂ | |
| 3.26 | Br | Br | -OCH₂CH₂ | -NH(CH₂-c-Pr) | |
| 3.27 | Cl | SO₂Et | -OCH₂CH₂- | 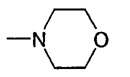 | |
| 3.28 | Cl | Cl | -OCH₂CH₂- | -N(Me)OMe | |
| 3.29 | Me | SO₂Et | -OCH₂CH₂- | -N(Me)OMe | |
| 3.30 | Br | Br | -OCH₂CH₂- | -N(Me)Ph | |
| 3.31 | Cl | Cl | -OCH₂CH=CH- | -NH₂ | |
| 3.32 | Br | Br | -OCH₂CH=CH- | -NH₂ | |
| 3.33 | Cl | Cl | -OCH₂CH=CH- | -NHMe | |
| 3.34 | Br | Br | -OCH₂CH=CH- | -NHMe | |
| 3.35 | Cl | Cl | -OCH₂CH=CH- | -NHEt | |
| 3.36 | Me | SO₂Me | -OCH₂CH=CH- | -NHEt | |
| 3.37 | Cl | Cl | -OCH₂CH=CH- | -NMe₂ | |
| 3.38 | Cl | SO₂Et | -OCH₂CH=CH- | -NMe₂ | |
| 3.39 | Cl | Cl | -OCH₂CH=CH- | -NEt₂ | |
| 3.40 | Cl | SO₂Et | -OCH₂CH=CH- | -NEt₂ | |
| 3.41 | Br | Br | -OCH₂CH=CH- | -NH(CH₂-c-Pr) | |
| 3.42 | Cl | SO₂Et | -OCH₂CH=CH- | 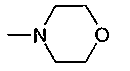 | |
| 3.43 | Cl | Cl | -OCH₂CH=CH- | -N(Me)OMe | |
| 3.44 | Me | SO₂Et | -OCH₂CH=CH- | -N(Me)OMe | |
| 3.45 | Br | Br | -OCH₂CH=CH- | -N(Me)Ph | |
| 3.46 | Cl | Cl | -OCH₂C≡C- | -NH₂ | |
| 3.47 | Br | Br | -OCH₂C≡C- | -NH₂ | |
| 3.48 | Cl | Cl | -OCH₂C≡C- | -NHMe | |
| 3.49 | Br | Br | -OCH₂C≡C- | -NHMe | |
| 3.50 | Cl | Cl | -OCH₂C≡C- | -NHEt | |
| 3.51 | Me | SO₂Me | -OCH₂C≡C- | -NHEt | |
| 3.52 | Cl | Cl | -OCH₂C≡C- | -NMe₂ | |
| 3.53 | Cl | SO₂Et | -OCH₂C≡C- | -NMe₂ | |
| 3.54 | Cl | Cl | -OCH₂C≡C- | -NEt₂ | |
| 3.55 | Cl | SO₂Et | -OCH₂C≡C- | -NEt₂ | |
| 3.56 | Br | Br | -OCH₂C≡C- | -NH(CH₂-c-Pr) | |
| 3.57 | Cl | SO₂Et | -OCH₂C≡C- | 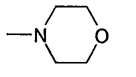 | |
| 3.58 | Cl | Cl | -OCH₂C≡C- | -N(Me)OMe | |
| 3.59 | Me | SO₂Et | -OCH₂C≡C- | -N(Me)OMe | |
| 3.60 | Br | Br | -OCH₂C≡C- | -N(Me)Ph | |
| 3.61 | Cl | SO₂Et | -OCH₂- | -NH₂ | R_{f} (EE): 0.02 |
| 3.62 | Cl | Cl | -OCH₂- | -NH(iso-propyl) | R_{f} (EE): 0.26 |
| 3.63 | Cl | Cl | -OCH₂- | -NH(CH₂CH=CH₂) | R_{f} (EE): 0.43 |
| 3.64 | Br | Br | -OCH₂- | -NMe₂ | R_{f} (EE): 0.07 |
| 3.65 | Br | Br | -OCH₂- | -NEt₂ | R_{f} (EE): 0.37 |
| 3.66 | Cl | Cl | -OCH₂- | -N(n-Prl)₂ | R_{f} (EE): 0.13 |
| 3.67 | Br | Br | -OCH₂- | -N(n-Pr)₂ | R_{f} (EE): 0.14 |
| 3.68 | Cl | SO₂Et | -OCH₂- | -N(n-Pr)₂ | R_{f} (EE): 0.06 |
| 3.69 | Cl | Cl | -OCH₂- | -N(i-Pr)₂ | R_{f} (EE): 0.15 |
| 3.70 | Br | Br | -OCH₂- | -N(i-Pr)₂ | R_{f} (EE): 0.17 |
| 3.71 | Cl | SO₂Et | -OCH₂- | -N(i-Pr)₂ | R_{f} (EE): 0.10 |
| 3.72 | Cl | Cl | -OCH₂- | -N(CH₂CH=CH₂)₂ | R_{f} (EE): 0.55 |
| 3.73 | Br | Br | -OCH₂- | -N(CH₂CH=CH₂)₂ | R_{f} (EE): 0.40 |
| 3.74 | Cl | Cl | -OCH₂- | -NMeEt | R_{f} (EE): 0.25 |
| 3.75 | Br | Br | -OCH₂- | -NMeEt | R_{f} (EE): 0.30 |
| 3.76 | Cl | SO₂Et | -OCH₂- | -NMeEt | R_{f} (EE): 0.21 |
| 3.77 | Cl | Cl | -OCH₂- | -NMe(CH₂Ph) | R_{f} (EE): 0.16 |
| 3.78 | Br | Br | -OCH₂- | -NMe(CH₂Ph) | R_{f} (EE): 0.14 |
| 3.79 | Cl | SO₂Et | -OCH₂- | -NMe(CH₂Ph) | R_{f} (EE): 0.09 |
| 3.80 | Cl | Cl | -OCH₂- | -N(Ph)Me | R_{f} (EE): 0.15 |
| 3.81 | Br | Br | -OCH₂- | -N(Ph)Me | R_{f} (EE): 0.14 |
| 3.82 | Cl | SO₂Et | -OCH₂- | -N(Ph)Me | R_{f} (EE): 0.08 |
| 3.83 | Cl | Cl | -OCH₂- | -N(Ph)(i-Pr) | R_{f} (EE): 0.18 |
| 3.84 | Br | Br | -OCH₂- | -N(Ph)(i-Pr) | R_{f} (EE): 0.18 |
| 3.85 | Cl | SO₂Et | -OCH₂- | -N(Ph)(i-Pr) | R_{f} (EE): 0.11 |
| 3.86 | Cl | Cl | -OCH₂- | 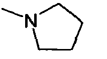 | R_{f} (EE): 0.06 |
| 3.87 | Br | Br | -OCH₂- | 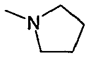 | R_{f} (EE): 0.04 |
| 3.88 | Cl | SO₂Et | -OCH₂- | 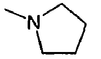 | R_{f} (EE): 0.02 |
| 3.89 | Cl | Cl | -OCH₂- | 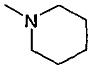 | R_{f} (EE): 0.06 |
| 3.90 | Br | Br | -OCH₂- | 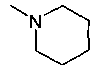 | R_{f} (EE): 0.08 |
| 3.91 | Cl | SO₂Et | -OCH₂- | 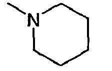 | R_{f} (EE): 0.09 |
| 3.92 | Cl | Cl | -OCH₂- | 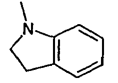 | R_{f} (EE): 0.17 |
| 3.93 | Br | Br | -OCH₂- | 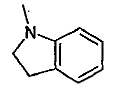 | R_{f} (EE): 0.17 |
| 3.94 | Cl | SO₂Et | -OCH₂- | 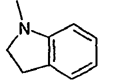 | R_{f} (EE): 0.08 |
| 3.95 | Cl | Cl | -OCH₂- | 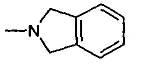 | R_{f} (EE): 0.09 |
| 3.96 | Br | Br | -OCH₂- | 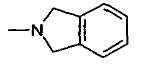 | R_{f} (EE): 0.10 |
| 3.97 | Cl | SO₂Et | -OCH₂- | 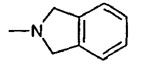 | R_{f} (EE): 0.06 |
| 3.98 | Br | Br | -OCH₂- | 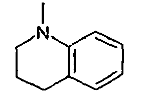 | R_{f} (EE): 0.15 |
| 3.99 | Cl | SO₂Et | -OCH₂- | 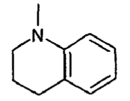 | R_{f} (EE): 0.12 |
| 3.100 | Cl | Cl | -OCH₂- | 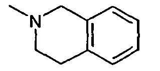 | R_{f} (EE): 0.13 |
| 3.101 | Cl | SO₂Et | -OCH₂- | 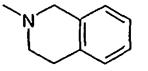 | R_{f} (EE): 0.04 |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man
75 Gew.-Teile einer Verbindung der allgemeinen Formel(I),
10 " ligninsulfonsaures Calcium,
5 " Natriumlaurylsulfat,
3 " Polyvinylalkohol und
7 " Kaolin
mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man
25 Gew.-Teile einer Verbindung der allgemeinen Formel (I),
5 " 2,2'-dinaphthylmethan-6,6'-disulfonsaures Natrium,
2 " oleoylmethyltaurinsaures Natrium,
1 " Polyvinylalkohol,
17 " Calciumcarbonat und
50 " Wasser
auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung im Vorauflauf

Samen von mono- und dikotylen Schadpflanzen werden in Papptöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 IIha in einer in Tabelle B1 angegebenen Dosierung auf die Oberfläche der Abdeckerde appliziert. Nach der Behandlung werden die Töpfe im Gewächshaus aufgestellt und unter guten Wachstumsbedingungen für die Schadpflanzen gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen. Wie die Ergebnisse dieser Tabelle zeigen, weisen die ausgewählten erfindungsgemäßen Verbindungen dabei eine hervorragende Wirksamkeit gegen ein breites Spektrum wirtschaftlich wichtiger mono- und dikotyler Schadpflanzen auf.

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 1/ha in einer in Tabelle B2 angegebenen Dosierung auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der Verbindungen im Vergleich zu Verbindungen, die im Stand der Technik offenbart sind, bonitiert. Wie die Ergebnisse dieser Tabelle zeigen, weisen die ausgewählten erfindungsgemäßen Verbindungen dabei eine hervorragende Wirksamkeit gegen ein breites Spektrum wirtschaftlich wichtiger mono- und dikotyler Schadpflanzen auf.

### 3. Kulturpflanzenverträglichkeit

In weiteren Versuchen im Gewächshaus werden Samen von Gerste und mono- und dikotyler Schadpflanzen in sandigem Lehmboden ausgelegt, mit Erde abgedeckt und im Gewächshaus aufgestellt, bis die Pflanzen zwei bis drei echte Blätter entwickelt haben. Die Behandlung mit den erfindungsgemäßen Verbindungen der Formel (I) erfolgt dann wie oben unter Punkt 2 beschrieben. Vier bis fünf Wochen nach der Applikation und Standzeit im Gewächshaus wird mittels optischer Bonitur festgestellt, daß die erfindungsgemäßen Verbindungen eine hervorragende Verträglichkeit gegenüber wichtigen Kulturpflanzen, insbesondere Weizen, Mais und Reis, aufweisen.

**Die in folgenden Vergleichstabellen verwendeten Abkürzungen bedeuten:**

| | | | |
|---|---|---|---|
| AMARE | Amaranthus retroflexus | CYPSE | Cyperus serotinus |
| MOOVA | Monochoria vaginalis | ORYSP | Oryza sativa |
| SAGPY | Sagittaria pygmaea | SINAL | Sinapis arvensis |
| STEME | Stellaria media | | |

**Tabelle B1, herbizide Wirkung im Vorauflauf**

| Verbindung Nr. | Dosierung [g a.i./ha] | Wirkung gegen Schadpflanzen | | |
|---|---|---|---|---|
| | | AMARE | SINAL | STETE |
| 1.28 | 320 | 90% | 90% | 90% |
| 3.9 | 320 | 90% | 80% | 90% |
| 3.65 | 320 | 90% | 80% | 90% |

**Tabelle B2, herbizide Wirkung im Nachauflauf**

| Verbindung Nr. | Dosierung [g a.i./ha] | Wirkung gegen Schadpflanzen | | |
|---|---|---|---|---|
| | | AMARE | SIGNAL | STEME |
| 3.9 | 320 | 80% | 90% | 100% |
| 3.65 | 320 | 80% | 90% | 100% |
| 3.87 | 320 | 70% | 80% | 90% |

**Tabelle B3, Wirkung im Nachauflauf**

| Verbindung Nr. | Dosierung [g a.i./ha] | Schädigung der Kulturpflanzen ORYSP | Wirkung gegen Schadpflanzen | | |
|---|---|---|---|---|---|
| | | | CYPSE | MOOVA | SAGPY |
| 3.9 | 90 | 0% | 95% | 95% | 90% |
| 3.65 | 50 | 0% | 99% | 99% | 80% |

## Patentansprüche

1. Verbindungen der Formel (I) oder deren Salze 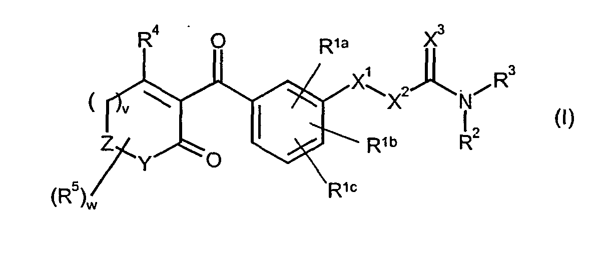 worin
X¹ bedeutet eine divalente Einheit aus der Gruppe O, S(O)ₙ, N-H, N-R²;
X² bedeutet eine geradkettige oder verzweigte durch w Reste aus der Gruppe Halogen, Cyano und Nitro und durch v Reste R² substituierte (C₁-C₆)-Alkylen-, (C₂-C₆)-Alkenylen- oder (C₂-C₆)-Alkinylenkette;
X³ bedeutet Sauerstoff oder Schwefel;
R^{1a}, R^{1b}, R^{1c} bedeuteten unabhängig voneinander Wasserstoff, Mercapto, Nitro, Halogen, Cyano, Thiocyanato, (C₁-C₆)-Alkyl-CO-O, (C₁-C₆)-Alkyl-S(O)ₙ-O, (C₁-C₆)-Alkyl-S(O)ₙ, Di-(C₁-C₆)-alkyl-NH-SO₂, (C₁-C₆)-Alkyl-SO₂-NH, (C₁-C₆)-Alkyl-NH-CO, (C₁-C₆)-Alkyl-SO₂-[(C₁-C₆)-alkyl]amino, (C₁-C₆)-Alkyl-CO-[(C₁-C₆)-alkyl]amino, 1,2,4-Triazol-1-yl, (C₁-C₆)-Alkyl-O-CH₂, (C₁-C₆)-Alkyl-S(O)ₙ-CH₂, (C₁-C₆)-Alkyl-NH-CH₂, 1,2,4-Triazol-1-yl-CH₂, jeweils durch v Reste aus der Gruppe Cyano, Nitro und Halogen substituiertes (C₁-C₆)-Alkyl-(D)ₚ, (C₂-C₆)-Alkenyl-(D)ₚ, (C₂-C₆)-Alkinyl-(D)ₚ, (C₃-C₉)-Cycloalkyl-(D)ₚ, (C₃-C₉)-Cyloalkenyl-(D)ₚ, (C₁-C₆)-Alkyl-Cycloalkyl-(D)ₚ, (C₁-C₆)-Alkyl-Cycloalkenyl-(D)ₚ;
D bedeutet Sauerstoff oder Schwefel;
R², R³ bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₃-C₉)-Cycloalkenyl, (C₁-C₆)-Alkyl-(C₃-C₉)-Cycloalkyl, (C₁-C₆)-Alkyl-(C₃-C₉)-Cycloalkenyl, (C₂-C₆)-Alkenyl-(C₃-C₉)-Cycloalkyl, (C₂-C₆)-Alkenyl-(C₃-C₉)-Cycloalkenyl, (C₂-C₆)-Alkinyl-(C₃-C₉)-Cycloalkyl, (C₂-C₆)-Alkinyl-(C₃-C₉)-Cycloalkenyl, geradkettiges oder verzweigtes [O-C(R⁶)₂]_{w}-[O-C(R⁶)-₂]ₓ-R⁶, (C₁-C₆)-Alkyl-aryl, (C₂-C₆)-Alkenyl-aryl, (C₂-C₆)-Alkinyl-aryl, geradkettiges oder verzweigtes [O-C(R⁶)₂]_{w}-[O-C(R⁶)₂]ₓ-Aryl, wobei die vorstehend genannten kohlenstoffhaltigen Reste durch v Reste aus der Gruppe Cyano, Nitro und Halogen substituiert sind, durch v Reste aus der Gruppe Cyano, Nitro, Halogen, (C₁-C₆)-Alkyl-(D)p und Halogen-(C₁-C₆)-alkyl-(D)ₚ substituiertes Aryl, Heterocyclyl oder Heteroaryl,
oder
R² und R³ bilden gemeinsam mit dem sie bindenden Stickstoffatom einen 5- oder 6- gliedrigen, gesättigten, teilweise oder vollständig ungesättigten Ring enthaltend m Heteroatome aus der Gruppe Sauerstoff und Stickstoff, an den optional ein Phenylring benzokondensiert ist, wobei der 5- oder 6-gliedrige Ring durch v Reste aus der Gruppe Cyano, Nitro, Halogen, (C₁-C₆)-Alkyl-(D)ₚ und Halogen-(C₁-C₆)-alkyl-(D)ₚ substituiert ist und wobei der ankondensierte Phenylring durch v Reste aus der Gruppe Cyano, Nitro und Halogen substituiert ist;
R⁴ bedeutet OR⁷, (C₁-C₄)-Alkylthio, Halogen-(C₁-C₄)-alkylthio, (C₂-C₄)-Alkenylthio, Halogen-(C₂-C₄)-alkenylthio, (C₂-C₄)-Alkinylthio, Halogen-(C₂-C₄)-alkinylthio, (C₁-C₄)-Alkylsulfinyl, Halogen-(C₁-C₄)-alkylsulfinyl, (C₂-C₄)-Alkenylsulfinyl, Halogen-(C₂-C₄)-alkenylsulfinyl, (C₂-C₄)-Alkinylsulfinyl, Halogen-(C₂-C₄)-alkinylsulfinyl, (C₁-C₄)-Alkylsulfonyl, Halogen-(C₁-C₄)-alkylsulfonyl, (C₂-C₄)-Alkenylsulfonyl, Halogen-(C₂-C₄)-alkenylsulfonyl, (C₂-C₄)-Alkinylsulfonyl, Halogen-(C₂-C₄)-alkinylsulfonyl, Halogen, Cyano, Cyanato, Thiocyanato oder Phenylthio;
R⁵ bedeutet Wasserstoff, Tetrahydropyran-3-yl, Tetrahydropyran-4-yl, Tetrahydrothiopyran-3-yl, (C₁-C₄)-Alkyl, (C₁-C₈)-Cycloalkyl, (C₁-C₄)-Alkoxy, (C₁-C₄)-Alkoxy-(C₁-C₄)-Alkyl, (C₁-C₄)-Alkoxycarbonyl, (C₁-C₄)-Alkylthio, Phenyl, wobei die acht letztgenannten Gruppen durch v Reste aus der Gruppe Halogen, (C₁-C₄)-Alkylthio und (C₁-C₄)-Alkoxy substituiert sind,
oder
zwei an einem gemeinsamen C-Atom gebundene Reste R⁵ bilden eine Kette aus der Gruppe OCH₂CH₂O, OCH₂CH₂CH₂O, SCH₂CH₂S und SCH₂CH₂CH₂S, wobei diese durch w Methylengruppen substituiert ist, oder zwei an direkt benachbarten C-Atome gebundene Reste R⁵ bilden mit den sie tragenden C-Atomen einen durch w Reste aus der Gruppe Halogen, (C₁-C₄)-Alkyl, (C₁-C₄)-Alkylthio und (C₁-C₄)-Alkoxy substituierten, 3- bis 6-gliedrigen Ring;
R⁶ bedeutet Wasserstoff, Halogen, Cyano oder Nitro, (C₁-C₄)-Alkyl, Halogen-(C₁-C₄)-alkyl;
R⁷ bedeutet Wasserstoff, (C₁-C₄)-Alkyl, Halogen-(C₁-C₄)-alkyl, (C₁-C₄)-Alkoxy-(C₁-C₄)-alkyl, Formyl, (C₁-C₄)-Alkylcarbonyl, (C₁-C₄)-Alkoxycarbonyl, (C₁-C₄)-Alkylaminocarbonyl, Di-(C₁-C₄)-alkylaminocarbonyl, (C₁-C₄)-Alkylsulfonyl, Halogen-(C₁-C₄)-alkylsulfonyl, Phenyl, Benzoyl oder Phenylsulfonyl, wobei die drei letzgenannten Gruppen durch v Reste aus der Gruppe (C₁-C₄)-Alkyl, Halogen-(C₁-C₄)-alkyl, (C₁-C₄)-Alkoxy, Halogen-(C₁-C₄)-alkoxy, Halogen, Cyano und Nitro substituiert sind;
Y bedeutet eine divalente Einheit aus der Gruppe O, S, N-H, N-(C₁-C₆)-Alkyl, CHR⁵ und C(R⁵)₂;
Z bedeutet eine direkte Bindung oder eine divalente Einheit aus der Gruppe O, S, SO, SO₂, N-H, N-Alkyl, CHR⁶ oder C(R⁶)₂;
m und n bedeuten jeweils unabhängig voneinander 0, 1 oder 2;
p bedeutet jeweils unabhängig voneinander 0 oder 1;
v bedeutet 0, 1, 2 oder 3;
w und x bedeuten jeweils unabhängig voneinander 0,1, 2, 3 oder 4,
wobei w und x nicht gleichzeitig null sein sollen.

2. Verbindungen nach Anspruch 1, worin
R², R³ bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₃-C₉)-Cycloalkenyl, (C₁-C₆)-Alkyl-(C₃-C₉)-Cycloalkyl, (C₁-C₆)-Alkyl-(C₃-C₉)-Cycloalkenyl, (C₂-C₆)-Alkenyl-( C₃-C₉)-Cycloalkyl, (C₂-C₆)-Alkenyl-(C₃-C₉)-Cycloalkenyl, (C₂-C₆)-Alkinyl-(C₃-C₉)-Cycloalkyl, (C₂-C₆)-Alkinyl-(C₃-C₉)-Cycloalkenyl, geradkettiges oder verzweigtes [O-C(R⁶)₂]_{w}-[O-C(R⁶)-₂]ₓ-R⁶, wobei die letzten 12 genannten Reste durch v Reste aus der Gruppe Cyano, Nitro und Halogen substituiert sind, durch v Reste aus der Gruppe Cyano, Nitro, Halogen, (C₁-C₆)-Alkyl-(D)p und Halogen-(C₁-C₆)-alkyl-(D)ₚ substituiertes Aryl,
oder
R² und R³ bilden gemeinsam mit dem sie bindenden Stickstoffatom einen 5- oder 6- gliedrigen, gesättigten, teilweise oder vollständig ungesättigten Ring enthaltend m Heteroatome aus der Gruppe Sauerstoff und Stickstoff, und der durch v Reste aus der Gruppe Cyano, Nitro, Halogen, (C₁-C₆)-Alkyl-(D)ₚ und Halogen-(C₁-C₆)-alkyl-(D)ₚ substituiert ist;
R⁷ bedeutet Wasserstoff, (C₁-C₄)-Alkylsulfonyl, Halogen-(C₁-C₄)-alkylsulfonyl, Phenyl, Benzoyl oder Phenylsulfonyl, wobei die drei letzgenannten Gruppen durch v Reste aus der Gruppe (C₁-C₂)-Alkyl, Halogen-(C₁-C₂)-alkyl, (C₁-C₂)-Alkoxy, Halogen-(C₁-C₂)-alkoxy, Halogen, Cyano und Nitro substituiert sind;
Y bedeutet eine divalente Einheit aus der Gruppe O, N-H, N-(C₁-C₆)-Alkyl, CHR⁵ und C(R⁵)₂, und
Z bedeutet eine divalente Einheit aus der Gruppe O, S, SO₂, (C₁-C₆)-Alkyl, CHR⁶ oder C(R⁶)₂.

3. Verbindungen nach Anspruch 1 oder 2, worin
X³ bedeutet Sauerstoff;
R^{1c} bedeutet Wasserstoff, und
R⁶ bedeutet Wasserstoff, (C₁-C₄)-Alkyl oder Halogen-(C₁-C₄)-alkyl.

4. Verbindungen nach einem der Ansprüche 1 bis 3, worin
X¹ bedeutet Sauerstoff;
R^{1a} und R^{1b} bedeuten jeweils Brom, Chlor, Fluor, Methyl, Methylthio, Methoxy, Methylsulfonyl, Ethylsulfonyl oder Trifluormethyl, und
R², R³ bedeuten unabhängig voneinander Wasserstoff, (C₁-C₆)-Alkyl, (C₂-C₆)-Alkenyl, (C₂-C₆)-Alkinyl, (C₃-C₉)-Cycloalkyl, (C₁-C₆)-Alkyl-(C₃-C₉)-cycloalkyl, geradkettiges oder verzweigtes [O-C(R⁶)₂]_{w}-[O-C(R⁶)-₂]ₓ-R⁶, wobei die letzten 6 genannten Reste durch v Reste aus der Gruppe Cyano, Nitro und Halogen substituiert sind, durch v Reste aus der Gruppe Cyano, Nitro, Halogen, (C₁-C₆)-Alkyl-(D)ₚ und Halogen-(C₁-C₆)-alkyl-(D)ₚ substituiertes Aryl, oder
R² und R³ bilden gemeinsam mit dem sie bindenden Stickstoffatom einen 5- oder 6- gliedrigen, gesättigten, teilweise oder vollständig ungesättigten Ring enthaltend m Heteroatome aus der Gruppe Sauerstoff und Stickstoff, und der durch v Reste aus der Gruppe Cyano, Nitro, Halogen, (C₁-C₆)-Alkyl-(D)ₚ und Halogen-(C₁-C₆)-alkyl-(D)ₚ substituiert ist.

5. Verbindungen nach einem der Ansprüche 1 bis 4, worin
R⁴ bedeutet OR⁷, (C₁-C₄)-Alkylthio, (C₂-C₄)-Alkenylthio, (C₁-C₄)-Alkylsulfonyl, Halogen, Cyano, Cyanato, Thiocyanato oder Phenylthio und
R⁵ bedeutet Wasserstoff, (C₁-C₄)-Alkyl, (C₁-C₈)-Cycloalkyl, (C₁-C₄)-Alkoxy, (C₁-C₄)-Alkylthio, Phenyl, oder zwei an direkt benachbarten C-Atomen gebundene Reste R⁵ bilden mit den sie tragenden C-Atomen einen substituierten 3- bis 6-gliedrigen Ring.

6. Verbindungen nach einem der Ansprüche 1 bis 5, worin der Substituent R^{1a} in 2-Position und der Substituent R^{1b} in 4-Position des Phenylrings steht.

7. Verbindungen nach einem der Ansprüche 1 bis 6, worin
R⁴ bedeutet OR⁷;
D bedeutet Sauerstoff;
Y und Z bedeuten die Gruppe CH₂, und
v und w bedeuten jeweils unabhängig voneinander 0, 1 oder 2.

8. Verbindungen nach einem der Ansprüche 1 bis 7, worin diese Verbindungen nicht als Salz vorliegen.

9. Herbizide Mittel, **gekennzeichnet durch** einen herbizid wirksamen Gehalt an mindestens einer Verbindung der allgemeinen Formel (I) gemäß einem der Ansprüche 1 bis 8.

10. Herbizide Mittel nach Anspruch 9 in Mischung mit Formulierungshilfsmitteln.

11. Verfahren zur Bekämpfung unerwünschter Pflanzen, **dadurch gekennzeichnet, daß** man eine wirksame Menge mindestens einer Verbindung der allgemeinen Formel (I) nach einem der Ansprüche 1 bis 8 oder eines herbiziden Mittels nach Anspruch 9 oder 10 auf die Pflanzen oder auf den Ort des unerwünschten Pflanzenwachstums appliziert.

12. Verwendung von Verbindungen der allgemeinen Formel (I) nach einem der Ansprüche 1 bis 8 oder von herbiziden Mitteln nach Anspruch 9 oder 10 zur Bekämpfung unerwünschter Pflanzen.

13. Verwendung nach Anspruch 12, **dadurch gekennzeichnet, daß** die Verbindungen der allgemeinen Formel (I) zur Bekämpfung unerwünschter Pflanzen in Kulturen von Nutzpflanzen eingesetzt werden.

14. Verwendung nach Anspruch 13, **dadurch gekennzeichnet, daß** die Nutzpflanzen transgene Nutzpflanzen sind.

## Claims

1. A compound of the formula (I) or a salt thereof 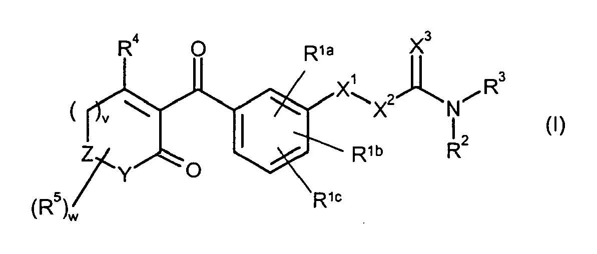 in which
X¹ is a divalent unit selected from the group consisting of O, S(O)ₙ, N-H and N-R²;
X² is a straight-chain or branched (C₁-C₆)-alkylene, (C₂-C₆)-alkenylene or (C₂-C₆)-alkynylene chain which is substituted by w radicals selected from the group consisting of halogen, cyano and nitro and by v radicals R²;
X³ is oxygen or sulfur;
R^{1a}, R^{1b}, R^{1c} independently of one another are hydrogen, mercapto, nitro, halogen, cyano, thiocyanato, (C₁-C₆)-alkyl-CO-O, (C₁-C₆)-alkyl-S(O)ₙ-O, (C₁-C₆)-alkyl-S(O)ₙ, di-(C₁-C₆)-alkyl-NH-SO₂, (C₁-C₆)-alkyl-SO₂-NH, (C₁-C₆)-alkyl-NH-CO, (C₁-C₆)-alkyl-SO₂-[(C₁-C₆)-alkyl]amino, (C₁-C₆)-alkyl-CO-[(C₁-C₆)-alkyl]amino, 1,2,4-triazol-1-yl, (C₁-C₆)-alkyl-O-CH₂, (C₁-C₆)-alkylS(O)ₙ-CH₂, (C₁-C₆)-alkyl-NH-CH₂, 1,2,4-triazol-1-yl-CH₂, or are (C₁-C₆)-alkyl-(D)ₚ, (C₂-C₆)-alkenyl-(D)ₚ, (C₂-C₆)-alkynyl-(D)ₚ, (C₃-C₉)-cycloalkyl-(D)ₚ, (C₃-C₉)-cycloalkenyl-(D)ₚ, (C₁-C₆)-alkyl-cycloalkyl-(D)ₚ, (C₁-C₆)-alkyl-cycloalkenyl-(D)ₚ, each of which is substituted by v radicals selected from the group consisting of cyano, nitro and halogen;
D is oxygen or sulfur;
R², R³ independently of one another are hydrogen, (C₁-C₆)-alkyl, (C₂-C₆)-alkenyl, (C₂-C₆)-alkynyl, (C₃-C₉)-cycloalkyl, (C₃-C₉)-cycloalkenyl, (C₁-C₆)-alkyl-(C₃-C₉)-cycloalkyl, (C₁-C₆)-alkyl-(C₃-C₉)-cycloalkenyl, (C₂-C₆)-alkenyl-(C₃-C₉)-cycloalkyl, (C₂-C₆)-alkenyl-(C₃-C₉)-cycloalkenyl, (C₂-C₆)-alkynyl-(C₃-C₉)-cycloalkyl, (C₂-C₆)-alkynyl-(C₃-C₉)-cycloalkenyl, straight-chain or branched [O-C(R⁶)₂]_{w}-[O-C(R⁶)₂]ₓ-R⁶, (C₁-C₆)-alkylaryl, (C₂-C₆)-alkenylaryl, (C₂-C₆)-alkynylaryl, straight-chain or branched [O-C(R⁶)₂]_{w}-[O-C(R⁶)₂]ₓ-aryl, the abovementioned carbon-containing radicals being substituted by v radicals selected from the group consisting of cyano, nitro and halogen, aryl, heterocyclyl or heteroaryl, each of which is substituted by v radicals selected from the group consisting of cyano, nitro, halogen, (C₁-C₆)-alkyl-(D)ₚ and halo-(C₁-C₆)-alkyl-(D)ₚ,
or
R² and R³ together with the nitrogen atom to which they are bonded form a 5- or 6-membered, saturated, partially or fully unsaturated ring comprising m hetero atoms selected from the group consisting of oxygen and nitrogen, the 5- or 6-membered ring optionally being benzo-fused to a phenyl ring and being substituted by v radicals selected from the group consisting of cyano, nitro, halogen, (C₁-C₆)-alkyl-(D)p and halO-(C₁-C₆)-alkyl-(D)p and the fused phenyl ring being substituted by v radicals selected from the group consisting of cyano, nitro and halogen;
R⁴ is OR⁷, (C₁-C₄)-alkylthio, halO-(C₁-C₄)-alkylthio, (C₂-C₄)-alkenylthio, halo-(C₂-C₄)-alkenylthio, (C₂-C₄)-alkynylthio, halo-(C₂-C₄)-alkynylthio, (C₁-C₄)-alkylsulfinyl, halo-(C₁-C₄)-alkylsulfinyl, (C₂-C₄)-alkenylsulfinyl, halo-(C₂-C₄)-alkenylsulfinyl, (C₂-C₄)-alkynylsulfinyl, halo-(C₂-C₄)-alkynylsulfinyl, (C₁-C₄)-alkylsulfonyl, halo-(C₁-C₄)-alkylsulfonyl, (C₂-C₄)-alkenylsulfonyl, halo-(C₂-C₄)-alkenylsulfonyl, (C₂-C₄)-alkynylsulfonyl, halo-(C₂-C₄)-alkynylsulfonyl, halogen, cyano, cyanato, thiocyanato or phenylthio;
R⁵ is hydrogen, tetrahydropyran-3-yl, tetrahydropyran-4-yl, tetrahydrothiopyran-3-yl, (C₁-C₄)-alkyl, (C₁-C₈)-cycloalkyl, (C₁-C₄)-alkoxy, (C₁-C₄)-alkoxy-(C₁-C₄)-alkyl, (C₁-C₄)-alkoxycarbonyl, (C₁-C₄)-alkylthio, phenyl, the eight last-mentioned groups being substituted by v radicals selected from the group consisting of halogen, (C₁-C₄)-alkylthio and (C₁-C₄)-alkoxy,
or
two radicals R⁵ bonded to a joint carbon atom form a chain selected from the group consisting of OCH₂CH₂O, OCH₂CH₂CH₂O, SCH₂CH₂S and SCH₂CH₂CH₂S, this chain being substituted by w methylene groups, or two radicals R⁵ bonded to directly adjacent carbon atoms, together with the carbon atoms bearing them, form a 3- to 6-membered ring which is substituted by w radicals selected from the group consisting of halogen, (C₁-C₄)-alkyl, (C₁-C₄)-alkylthio and (C₁-C₄)-alkoxy;
R⁶ is hydrogen, halogen, cyano or nitro, (C₁-C₄)-alkyl, halo-(C₁-C₄)-alkyl;
R⁷ is hydrogen, (C₁-C₄)-alkyl, halo-(C₁-C₄)-alkyl, (C₁-C₄)-alkoxy-(C₁-C₄)-alkyl, formyl, (C₁-C₄)-alkylcarbonyl, (C₁-C₄)-alkoxycarbonyl, (C₁-C₄)-alkylaminocarbonyl, di-(C₁-C₄)-alkylaminocarbonyl, (C₁-C₄)-alkylsulfonyl, halo-(C₁-C₄)-alkylsulfonyl, phenyl, benzoyl or phenylsulfonyl, the three last-mentioned groups being substituted by v radicals selected from the group consisting of (C₁-C₄)-alkyl, halo-(C₁-C₄)-alkyl, (C₁-C₄)-alkoxy, halo-(C₁-C₄)-alkoxy, halogen, cyano and nitro;
Y is a divalent unit selected from the group consisting of O, S, N-H, N-(C₁-C₆)-alkyl, CHR⁵ and C(R⁵)₂;
Z is a direct bond or a divalent unit selected from the group consisting of O, S, SO, SO₂, N-H, N-alkyl, CHR⁶ or C(R⁶)₂;
m and n in each case independently of one another are 0, 1 or 2;
p is independently 0 or 1;
v is 0, 1, 2 or 3;
w and x in each case independently of one another are 0, 1, 2, 3 or 4,
with the proviso that w and x are not simultaneously zero.

2. A compound as claimed in claim 1, in which
R², R³ independently of one another are hydrogen, (C₁-C₆)-alkyl, (C₂-C₆)-alkenyl, (C₂-C₆)-alkynyl, (C₃-C₉)-cycloalkyl, (C₃-C₉)-cycloalkenyl, (C₁-C₆)-alkyl-(C₃-C₉)-cycloalkyl, (C₁-C₆)-alkyl-(C₃-C₉)-cycloalkenyl, (C₂-C₆)-alkenyl-(C₃-C₉)-cycloalkyl, (C₂-C₆)-alkenyl-(C₃-C₉)-cycloalkenyl, (C₂-C₆)-alkynyl-(C₃-C₉)-cydoalkyl, (C₂-C₆)-alkynyl-(C₃-C₉)-cycloalkenyl, straight-chain or branched
[O-C(R⁶)₂]_{w}-[O-C(R⁶)₂]ₓ-R⁶, the 12 last-mentioned radicals being substituted by v radicals selected from the group consisting of cyano, nitro and halogen,
aryl which is substituted by v radicals selected from the group consisting of cyano, nitro, halogen, (C₁-C₆)-alkyl-(D)ₚ and halo-(C₁-C₆)-alkyl-(D)ₚ
or
R² and R³ together with the nitrogen atom to which they are bonded form a 5- or 6-membered, saturated, partially or fully unsaturated ring comprising m hetero atoms selected from the group consisting of oxygen and nitrogen, which ring is substituted by v radicals selected from the group consisting of cyano, nitro, halogen, (C₁-C₆)-alkyl-(D)ₚ and halo-(C₁-C₆)-alkyl-(D)ₚ;
R⁷ is hydrogen, (C₁-C₄)-alkylsulfonyl, halo-(C₁-C₄)-alkylsulfonyl, phenyl, benzoyl or phenylsulfonyl, the three last-mentioned groups being substituted by v radicals selected from the group consisting of (C₁-C₂)-alkyl, halo-(C₁-C₂)-alkyl, (C₁-C₂)-alkoxy, halo-(C₁-C₂)-alkoxy, halogen, cyano and nitro;
Y is a divalent unit selected from the group consisting of O, N-H, N-(C₁-C₆)-alkyl, CHR⁵ and C(R⁵)₂, and
Z is a divalent unit selected from the group consisting of O, S, SO₂, (C₁-C₆)-alkyl, CHR⁶ or C(R⁶)₂.

3. A compound as claimed in claim 1 or 2, in which
X³ is oxygen,
R^{1c} is hydrogen, and
R⁶ is hydrogen, (C₁-C₄)-alkyl or halo-(C₁-C₄)-alkyl.

4. A compound as claimed in any of claims 1 to 3, in which
X¹ is oxygen;
R^{1a} and R^{1b} are in each case bromine, chlorine, fluorine, methyl, methylthio, methoxy, methylsulfonyl, ethylsulfonyl or trifluoromethyl, and
R², R³ independently of one another are hydrogen, (C₁-C₆)-alkyl, (C₂-C₆)-alkenyl, (C₂-C₆)-alkynyl, (C₃-C₉)-cycloalkyl, (C₁-C₆)-alkyl-(C₃-C₉)-cycloalkyl, straight-chain or branched [O-C(R⁶)₂]_{w}-[O-C(R⁶)-₂]ₓ-R⁶, where the 6 last-mentioned radicals are substituted by v radicals selected from the group consisting of cyano, nitro and halogen, are aryl which is substituted by v radicals selected from the group consisting of cyano, nitro, halogen, (C₁-C₆)-alkyl-(D)ₚ and halo-(C₁-C₆)-alkyl-(D)ₚ,
or
R² and R³ together with the nitrogen atom to which they are bonded form a 5- or 6-membered, saturated, partially or fully unsaturated ring comprising m hetero atoms selected from the group consisting of oxygen and nitrogen, which ring is substituted by v radicals selected from the group consisting of cyano, nitro, halogen, (C₁-C₆)-alkyl-(D)ₚ and halo-(C₁-C₆)-alkyl-(D)ₚ.

5. A compound as claimed in any of claims 1 to 4, in which
R⁴ is OR⁷, (C₁-C₄)-alkylthio, (C₂-C₄)-alkenylthio, (C₁-C₄)-alkylsulfonyl, halogen, cyano, cyanato, thiocyanato or phenylthio and
R⁵ is hydrogen, (C₁-C₄)-alkyl, (C₁-C₈)-cycloalkyl, (C₁-C₄)-alkoxy, (C₁-C₄)-alkylthio, phenyl, or two radicals R⁵ bonded to directly adjacent carbon atoms together with the carbon atoms to which they are bonded form a substituted 3- to 6-membered ring.

6. A compound as claimed in any of claims 1 to 5, in which the substituent R^{1a} is in the 2-position and the substituent R^{1b} is in the 4-position of the phenyl ring.

7. A compound as claimed in any of claims 1 to 6, in which
R⁴ is OR⁷;
D is oxygen;
Y and Z are the group CH₂, and
v and w are in each case independently of one another 0, 1 or 2.

8. A compound as claimed in any of claims 1 to 7, which are not in salt form.

9. A herbicidal composition which comprises a herbicidally active content of at least one compound of the formula (I) as claimed in any of claims 1 to 8.

10. A herbicidal composition as claimed in claim 9 as a mixture with formulation auxiliaries.

11. A method of controlling undesired plants, which comprises applying an effective amount of at least one compound of the formula (I) as claimed in any of claims 1 to 8 or of a herbicidal composition as claimed in claim 9 or 10 to the plants or to the site with undesired vegetation.

12. The use of a compound of the formula (I) as claimed in any of claims 1 to 8 or of a herbicidal composition as claimed in claim 9 or 10 for controlling undesired plants.

13. The use as claimed in claim 12, wherein the compounds of the formula (I) are employed for controlling undesired plants in crops of useful plants.

14. The use as claimed in claim 13, wherein the useful plants are transgenic useful plants.

## Revendications

1. Composés de la formule (I) ou sels de ceux-ci 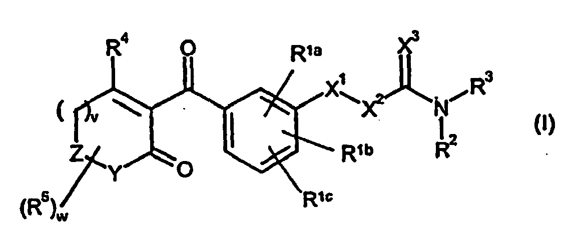 dans laquelle
X¹ représente une unité divalente choisie dans le groupe de O, S(O)ₙ, N-H, N-R² ;
X² représente une chaîne alkylène en C₁-C₆, alcénylène en C₂-C₆
ou alcynyline en C₂-C₆ linéaire ou ramifiée par w restes choisis dans le groupe constitué des groupes halogène cyano et nitro et substituée par v restes R² ;
X³ représente l'oxygène ou le soufre ;
R^{1a}, R^{1b}, R^{1c} représentent indépendamment les uns des autres l'hydrogène, un groupe mercapto, nitro, halogène, cyano, thiocyanato, (alkyle en C₁-C₆)-CO-O, (alkyle en C₁-C₆)-S(O)ₙ-O, (alkyle en C₁-C₆)-S(O)ₙ, Di-(alkyle en C₁-C₆)-NH-SO₂, (alkyle en C₁-C₆)-SO₂-NH, (alkyle en C₁-C₆)-NH-CO, (alkyle en C₁-C₆)-SO₂-[alkyle en C₁-C₆]amino, (alkyle en C₁-C₆)-CO-[alkyle en C₁-C₆]amino, 1,2,4-triazol-1-yle, (alkyle en C₁-C₆)-O-CH₂, (alkyle en C₁-C₆)-S(O)ₙ-CH₂, (alkyle en C₁-C₆)-NH-CH₂, 1,2,4-triazol-1-yl-CH₂, un groupe (alkyle en C₁-C₆)-(D)ₚ, (alcényle en C₂-C₆)-(D)ₚ, (alcynyle en C₂-C₆)-(D)ₚ, (cycloalkyle en C₃-C₉)-(D)ₚ, (cycloalcényle en C₃-C₉)-(D)ₚ, (alkyle en C₁-C₆)-cycloalkyle-(D)ₚ, (alkyle en C₁-C₆)-cycloalcényle-(D)ₚ substitué à chaque fois par v restes choisis dans le groupe constitué des groupes cyano, nitro et halogène ;
D représente l'oxygène ou le soufre ;
R², R³ représentent indépendamment l'un de l'autre l'hydrogène, un groupe alkyle en C₁-C₆, alcényle en C₂-C₆, alcynyle en C₂-C₆, cycloalkyle en C₃-C₉, cycloalcényle en C₃-C₉, (alkyle en C₁-C₆)-(cycloalkyle en C₃-C₉), (alkyle en C₁-C₆)-(cycloalcényle en C₃-C₉), (alcényle en C₂-C₆)-(cycloalkyle en C₃-C₉), (alcényle en C₂-C₆)-(cycloalcényle en C₃-C₉), (alcynyle en C₂-C₆)-(cycloalkyle en C₃-C₉), (alcynyle en C₂-C₆)-(cycloalcényle en C₃-C₉), [O-C(R⁶)₋₂]_{w}-[O-C(R₆)₋₂]ₓ-R⁶ linéaire ou ramifié, (alkyle en C₁-C₆)-aryle, (alcényle en C₂-C₆)-aryle, (alcynyle en C₂-C₆)-aryle, [O-C(R₆)₂]_{w}-[O-C(R⁶)₋₂]ₓ-aryle linéaire ou ramifié, les restes hydrocarbonés cités précédemment étant substitués par v restes choisis dans le groupe constitué des groupes cyano, nitro et halogène, et
un groupe aryle, hétérocyclyle ou hétéroaryle substitué par v restes choisis dans le groupe constitué des groupes cyano, nitro, halogène, (alkyle en C₁-C₆)-(D)ₚ et halogène-(alkyle en C₁-C₆)-(D)ₚ ou
R² et R³ forment ensemble avec l'atome d'azote les liant un noyau à 5 ou 6 éléments, saturé, partiellement ou totalement insaturé contenant m hétéroatomes choisis dans le groupe constitué des atomes d'oxygène et d'azote, sur lequel est éventuellement benzocondensé un noyau phényle, le noyau à 5 ou 6 éléments étant substitué par v restes choisis dans le groupe constitué des groupes cyano, nitro, halogène, (alkyle en C₁-C₆)-(D)ₚ et halogène-(alkyle en C₁-C₆)-(D)ₚ et le noyau phényle condensé étant substitué par v restes choisis dans le groupe constitué des groupes cyano, nitro et halogène ;
R⁴ représente OR⁷, un groupe alkylthio en C₁-C₄, halogène-(alkylthio en C₁-C₄)-, alcénylthio en C₂-C₄, halogène-(alcénylthio en C₂-C₄), alcynylthio en C₂-C₄, halogène-(alcynylthio en C₂-C₄), (alkyle en C₁-C₄)sulfinyle, halogène-(alkyle en C₁-C₄)sulfinyle, (alcényle en C₂-C₄)sulfinyle, halogène-(alcényle en C₂-C₄)sulfinyle, (alcynyle en C₂-C₄)sulfinyle, halogène-(alcynyle en C₂-C₄)sulfinyle, (alkyle en C₁-C₄)sulfonyle, halogène-(alkyle en C₁-C₄)sulfonyle, (alcényle en C₂-C₄)sulfonyle, halogène-(alcényle en C₂-C₄)sulfonyle, (alcynyle en C₂-C₄)sulfonyle, halogène-(alcynyle en C₂-C₄)sulfonyle, halogène, cyano, cyanato, thiocyanato ou phénylthio;
R⁵ représente l'hydrogène, un groupe tétrahydropyran-3-yle, tétrahydropyran-4-yle, tétrahydrothiopyran-3-yle, alkyle en C₁-C₄, cycloalkyle en C₁-C₈, alcoxy en C₁-C₄, (alcoxy en C₁-C₄)-(alkyle en C₁-C₄), (alcoxy en C₁-C₄)carbonyle, alkylthio en C₁-C₄, phényle, les 8 derniers groupes cités étant substitués par v restes choisis dans le groupe constitué des groupes halogène, alkylthio en C₁-C₄ et alcoxy en C₁-C₄,
ou
deux restes R⁵ liés à un atome C commun forment une chaîne choisie dans le groupe constitué de OCH₂CH₂O, OCH₂CH₂CH₂O, SCH₂CH₂S et SCH₂CH₂CH₂S, ceux-ci étant substitués par w groupes méthylène, ou deux restes R⁵ liés sur des atomes de C directement voisins forment avec l'atome C les portant un noyau à deux à trois éléments substitué par w restes choisis dans le groupe constitué des groupes halogène, alkyle en C₁-C₄, alkylthio en C₁-C₄ et alcoxy en C₁-C₄ ;
R⁶ représente l'hydrogène, un atome d'halogène, un groupe cyano ou nitro, alkyle en C₁-C₄, halogène-(alkyle en C₁-C₄) ;
R⁷ représente l'hydrogène, un groupe alkyle en C₁-C₄, halogène-(alkyle en C₁-C₄), (alcoxy en C₁-C₄)-(alkyle en C₁-C₄), formyle, alkylcarbonyle en C₁-C₄, alcoxycarbonyle en C₁-C₄, alkylaminocarbonyle en C₁-C₄, di-(alkylaminocarbonyle en C₁-C₄), alkylsulfonyle en C₁-C₄, halogène-(alkylsulfonyle en C₁-C₄), phényle, benzoyle ou phénylsulfonyle, les trois derniers groupes cités étant substitués par v restes choisis dans le groupe constitué des groupes alkyle en C₁-C₄, halogène-(alkyle en C₁-C₄), alcoxy en C₁-C₄, halogène-(alcoxy en C₁-C₄), halogène, cyano et nitro ;
Y représente une unité divalente choisie dans le groupe constitué de O, S, N-H, N-(alkyle en C₁-C₆), CHR⁵ et C(R⁵)₂ ;
Z représente une liaison directe ou une unité divalente choisie dans le groupe constitué de O, S, SO, SO₂, N-H, N-alkyle, CHR⁶ ou C(R⁶)₂ ;
m et n représentent chacun indépendamment l'un de l'autre 0, 1 ou 2 ;
p représente chacun indépendamment l'un de l'autre 0 ou 1 ;
v représente 0, 1, 2 ou 3 ;
w et x représentent chacun indépendamment l'un de l'autre 0, 1, 2, 3 ou 4, w et x ne devant pas être simultanément égaux à 0.

2. Composés selon la revendication 1, dans lesquels
R², R³ représentent indépendamment l'un de l'autre l'hydrogène, un groupe alkyle en C₁-C₆, alcényle en C₂-C₆, alcynyle en C₂-C₆, cycloalkyle en C₃-C₉, cycloalcényle en C₃-C₉, (alkyle en C₁-C₆)-(cycloalkyle en C₃-C₉), (alkyle en C₁-C₆)-(cycloalcényle en C₃-C₉), (alcényle en C₂-C₆)-(cycloalkyle en C₃-C₉), (alcényle en C₂-C₆)-(cycloalcényle en C₃-C₉), (alcynyle en C₂-C₆)-(cycloalkyle en C₃-C₉), (alcynyle en C₂-C₆)-(cycloalcényle en C₃-C₉), [O-C(R⁶)₂]_{w}[O-C(R⁶)-₂]ₓ-R⁶ linéaire ou ramifié les douze derniers restes cités étant substitués par v restes choisis dans le groupe constitué des groupes cyano, nitro et halogène,
un groupe aryle substitué par v restes choisis dans le groupe constitué des groupes cyano, nitro, halogène, (alkyle en C₁-C₆)-(D)ₚ et halogène-(alkyle en C₁-C₆)-(D)ₚ, ou
R² et R³ forment ensemble avec l'atome d'azote les liant un noyau à 5 ou 6 éléments, saturé, partiellement ou totalement insaturé contenant m hétéroatomes choisis dans le groupe constitué des atomes d'azote et d'oxygène, et lequel est substitué par v restes choisis dans le groupe constitué des groupes cyano, nitro, halogène, (alkyle en C₁-C₆)-(D)ₚ et halogène-(alkyle en C₁-C₆)-(D)ₚ ;
R⁷ représente l'hydrogène, un groupe alkylsulfonyle en C₁-C₄, halogène-(alkylsulfonyle en C₁-C₄), phényle, benzoyle ou phénylsulfonyle, les trois derniers groupes cités étant substitués par v restes choisis dans le groupe constitué des groupes alkyle en C₁-C₂, halogène-(alkyle en C₁-C₂), alcoxy en C₁-C₂, halogène-(alcoxy en C₁-C₂), halogène, cyano et nitro ;
Y représente une unité divalente choisie dans le groupe constitué de 0, N-H, N-(alkyle en C₁-C₆), CHR⁵ et C(R⁵)₂, et
Z représente une unité divalente choisie dans le groupe constitué de 0, S, SO₂, un groupe alkyle en C₁-C₆, CHR⁶ ou C(R⁶)₂.

3. Composés selon la revendication 1 ou 2, dans lesquels
X³ représente l'oxygène ;
R^{1c} représente l'hydrogène, et
R⁶ représente l'hydrogène, un groupe alkyle en C₁-C₄ ou halogène-(alkyle en C₁-C₄).

4. Composés selon l'une quelconque des revendications 1 à 3, dans lesquels
X¹ représente l'oxygène ;
R^{1a} et R^{1b} représentent chacun le brome, le chlore, le fluor, un groupe méthyle, méthylthio, méthoxy, méthylsulfonyle, éthylsulfonyle ou trifluorométhyle, et
R², R³ représentent indépendamment l'un de l'autre l'hydrogène, un groupe alkyle en C₁-C₆, alcényle en C₂-C₆, alcynyle en C₂-C₆, cycloalkyle en C₃-C₉, (alkyle en C₁-C₆)-(cycloalkyle en C₃-C₉), [O-C(R⁶)₂]_{w}-[O-C(R⁶)₋₂]ₓ-R⁶ linéaire ou ramifié, les six derniers restes cités étant substitués par v restes choisis dans le groupe constitué des groupes cyano, nitro et halogène, aryle substitué par v restes choisis dans le groupe constitué des groupes cyano, nitro, halogène, (alkyle en C₁-C₆)-(D)ₚ et halogène- (alkyle en C₁-C₆)-(D)ₚ ou
R² et R³ forment ensemble avec l'atome d'azote les liant un noyau à 5 ou 6 éléments, saturé, partiellement ou totalement insaturé contenant m hétéroatomes choisis dans le groupe constitué de l'oxygène et de l'azote, et lequel est substitué par v restes choisis dans le groupe constitué des groupes cyano, nitro, halogène, (alkyle en C₁-C₆)-(D)ₚ et halogène-(alkyle en C₁-C₆)-(D)ₚ.

5. Composés selon l'une quelconque des revendications 1 à 4, dans lesquels
R⁴ représente OR⁷, un groupe alkylthio en C₁-C₄, alcénylthio en C₂-C₄, alkylsulfonyle en C₁-C₄, halogène, cyano, cyanato, thiocyanato ou phénylthio, et
R⁵ représente l'hydrogène, un groupe alkyle en C₁-C₄, cycloalkyle en C₁-C₈, alcoxy en C₁-C₄, alkylthio en C₁-C₄, phényle, ou deux restes R⁵ liés à des atomes C directement voisins forment avec l'atome C les portant un noyau substitué à de 3 à 6 éléments.

6. Composés selon l'une quelconque des revendications 1 à 5, dans lesquels le substituant R^{1a} est en position 2 et le substituant R^{1b} est en position 4 du noyau phényle.

7. Composés selon l'une quelconque des revendications 1 à 6, dans lesquels
R⁴ représente OR⁷ ;
D représente l'oxygène ;
Y et Z représentent le groupe CH₂, et
v et w représentent chacun indépendamment l'un de l'autre 0, 1 ou 2.

8. Composés selon l'une quelconque des revendications 1 à 7, dans lesquels ces composés ne sont pas présents dans la forme de sel.

9. Agents herbicides **caractérisés par** une teneur efficace d'herbicide en au moins un composé de la formule générale (I) selon l'une quelconque des revendications 1 à 8.

10. Agents herbicides selon la revendication 9 en mélange avec des auxiliaires de formulation.

11. Procédé pour combattre des plantes indésirables, **caractérisé en ce que** l'on applique une quantité efficace d'au moins un composé de la formule générale (I) selon l'une quelconque des revendications 1 à 8 ou d'un agent herbicide selon la revendication 9 ou 10 sur les plantes ou à l'endroit de croissance des plantes indésirables.

12. Utilisation de composés de la formule générale (I) selon l'une quelconque des revendications 1 à 8 ou d'agents herbicides selon la revendication 9 ou 10 pour combattre des plantes indésirables.

13. Utilisation selon la revendication 12, **caractérisée en ce que** l'on utilise les composés de la formule générale (I) pour combattre des plantes indésirables dans des cultures de plantes utiles.

14. Utilisation selon la revendication 13, **caractérisée en ce que** les plantes utiles sont des plantes utiles transgéniques.
